# EUROPEAN PATENT APPLICATION

(11) **EP 3 597 735 A1**
(43) Date of publication of application: **22.01.2020**
(21) Application number: 17900915.4
(22) Date of filing: 15.03.2017
(51) Int. Cl.: C12N 5/20, C07K 16/28, C12N 15/13, A61K 39/395, A61P 35/00, A61P 37/00

(54) **CTLA4 ANTIBODY, PHARMACEUTICAL COMPOSITION AND USE THEREOF**

(71) Applicant: Suzhou Galaxy Biopharma, Co., Ltd., Suzhou, Jiangsu 215028 (CN)
(72) Inventor: ZHOU, Honglin, Suzhou Jiangsu 215028 (CN); DONG, Xin, Suzhou Jiangsu 215028 (CN); LIU, Jie, Suzhou Jiangsu 215028 (CN); CAI, Bin, Suzhou Jiangsu 215028 (CN)
(74) Representative: Serjeants LLP
(86) International application number: PCT/CN2017/076751
(87) International publication number: WO 2018/165895

(57) **Abstract**

Provided is an isolated CTLA4 monoclonal antibody. The antibody can highly-specifically bind to CTLA4, and can effectively block the binding of CLTA4 to B7, reduce the activity or expression level of CTLA4, relieve the inhibition of CTLA4 on the immune function of an organism, activate T lymphocytes, and effectively treat tumours and immune system diseases. Also provided are a monoclonal antibody conjugate comprising the pharmaceutical composition and diagnostic composition of the antibody, and the use thereof in the preparation of a drug for preventing and/or treating and/or treating in combination tumours and immune system diseases.

## Description

### TECHNICAL FIELD

The present invention is in the fields of tumor immunotherapy and molecular immunology and relates to a CTLA4 antibody, the pharmaceutical composition and use thereof. In specific, the present invention relates to various monoclonal antibodies of CTLA4.

### BACKGROUND

The vertebrate immune system requires interactions of molecules and cells at multiple levels to achieve the optimal immune responses. Specifically, the activation of T lymphocytes (T cells) plays an important role in the immune response system of an organism. Antigen-presenting cells (APCs) can activate T cells by presenting antigens via peptides carried by major histocompatibility complex (MHC) to T cell receptors (TCRs). This activation also requires co-stimulation of APCs. Delivery of a non-specific co-stimulatory signal to T cells requires at least two homologous B7 family members found on APCs, *i.e.* B7-1 (also called B7, B7.1, or CD80) and B7-2 (also called B7.2 or CD86), both of which can deliver the co-stimulatory signals when bind to the CD28 receptor on T cells, resulting in the activation of T cells. CD28 is a homodimeric glycoprotein member of the immunoglobulin (Ig) superfamily having a single extracellular variable region, and is expressed on most mature human T cells.

Cytotoxic T lymphocyte-associated antigen-4, or CTLA4 for short, is very closely related to CD28 molecules in terms of gene structure, chromosome mapping, sequence homology, and genetic expression. Both CTLA4 and CD28 are the receptors of co-stimulatory molecules B7, and they are mainly expressed on the surfaces of activated T-cells. However, as a co-stimulatory signal for lymphocyte activation, the functions of CTLA4 and CD28 molecules are opposite. The role of CTLA4 is mainly to inhibit T cell activation, which is indicated by massive lymphadenia observed in CTLA4-deficient mice.

Previous studies indicate that the neutralizing antibody against CTLA4 or exogenous CTLA4 protein can block the binding of CTLA4 to the natural ligands thereof, thereby blocking the negative regulatory signal of CTLA4 to T cells and enhancing the reactivity of T cells to various antigens. Therefore, blocking CTLA4 may provide a novel method to treat relevant human diseases which require enhancement of immunostimulation, such as cancers and infectious diseases. Currently CTLA4 antibody has been approved for the treatment of malignant melanoma, and is used in clinical trials for prostate cancer, bladder cancer, colorectal cancer, gastrointestinal cancer, hepatic cancer, etc.

Interleukin 2 (IL-2) is a cytokine produced by T cells to regulate the T cells subpopulations, and is also an important factor for regulating immune response. IL-2 can also facilitate the proliferation and differentiation of B cells, participate in antibody responses, hemopoiesis and oncological surveillance. *In vitro* studies have shown that, CTLA4 antibody can specifically relieve the immunosuppression of CTLA4 to organisms, activate T cells and induce IL-2 production; and shows great prospect in the treatment of tumors and infectious diseases.

At present, it is still necessary to develop a new antibody and the humanized antibody thereof for blocking the binding of CTLA4 to B7.

### SUMMARY OF THE INVENTION

The present inventors use the recombinant CTLA4 protein expressed by mammalian cell expression system as the antigen to immunize mice. Hybridoma cells are obtained by fusion of mouse spleen cells and myeloma cells. After high-throughput screening for the samples, the inventors found several hybridoma cell strains which can secrete the specific monoclonal antibodies specifically binding to CTLA4, and the monoclonal antibodies can very effectively block the binding of CTLA4 to B7-1 or B7-2 (Figure 3, Table II). The chimeric antibodies were further obtained by means of molecular cloning and the like, and a series of studies on characterization and functional studies were carried out. The resultant chimeric antibodies can bind to CTLA4 on the surface of human T cells (Figure 4) with high affinity (Table V), at the same or different epitopes in comparison to the reference antibody (Figure 5). The *in vitro* functional test revealed that these chimeric antibodies can facilitate the immune function of T cell lines and primary T cells (Figures 7, 8); and the results of animal studies showed that these chimeric antibodies can effectively inhibit tumor growth in the humanized CTLA4 knock-in mice (Figure 9). Finally, these antibodies were humanized to reduce immunogenicity and to obtain the therapeutic antibody. The invention below was provided thereby.

The present invention provides an antibody that specifically binds to human CTLA4, and also provides chimeric and humanized antibodies in which the binding of human CTLA4 may inhibit the binding of human CTLA4 to human B7. The present invention also provides chimeric antibodies that specifically bind to human CTLA4 that block binding of human CTLA4 to human B7 by at least 10%, 20%, 30%, 40%, 50%, 60%, 70%, 80%, 90%, 99%, or 100%.

The present invention provides an antibody that specifically binds to human CTLA4, which was produced from hybridoma cell lines. The hybridoma cell lines provided by the present invention, which is derived from immunized mice and cell fusion, may produce monoclonal antibodies specifically blocking the binding of CTLA4 to CD80 through a series of operations such as screening. (See Example 1)

In one aspect, the present invention provides a murine monoclonal antibody to bind to the recombinant CTLA4 protein, and the EC₅₀ is in the range of 0.02 nM and 0.06 nM; the murine monoclonal antibody provided binds to the natural CTLA4 protein on the cell surface, and the EC₅₀ is in the range of 1 nM and 4 nM. In which, the term EC₅₀ refers to concentration for 50% of maximal effect. (See Example 2)

In another aspect of the present invention, murine monoclonal antibody is provided to block the binding of CTLA4 to B7-1 (CD80) or B7-2 (CD86), with IC₅₀ in the ranges of 2 ng/mL to 70 ng/mL and 200 ng/mL to 11000 ng/mL, respectively. In which, IC₅₀ (half maximal inhibitory concentration) refers to half inhibitory concentration of the antagonist being measured. (See Example 3)

Unless otherwise stated, the B7 in the present invention is B7-1 and/or B7-2; and the specific protein sequences are known sequences in the prior art, which can be referred to the sequences disclosed in the existing literatures or *GenBank.* For example, B7-1 (CD80, NCBI Gene ID: 941); B7-2 (CD86, NCBI Gene ID: 942).

In one aspect, the murine monoclonal antibody provided in the present invention shows weaker species cross reaction with mice but has species cross reaction with cynomolgus monkeys. (See Example 4)

The present invention also provides chimeric antibodies that specifically bind to human CTLA4, wherein the heavy and light chains of the chimeric antibody are constructed by connecting the variable region of the murine CTLA4 antibody to the constant regions of human IgG1 and kappa. The chimeric antibodies provided by the invention have similar affinity as compared to murine antibodies. (See Example 5)

In one aspect, the present invention provides an isolated anti-CTLA4 antibody or antigen-binding fragments thereof inhibiting the binding of CTLA4 to human B7, the isolated anti-CTLA4 antibody or antigen-binding fragments thereof comprise heavy and light chain variable regions, wherein:
the heavy chain variable region comprises a heavy chain CDR1 as set forth in SEQ ID NO: 13, a heavy chain CDR2 as set forth in SEQ ID NO: 14, and a heavy chain CDR3 as set forth in SEQ ID NO: 15; and/or the light chain variable region comprises a light chain CDR1 as set forth in SEQ ID NO: 16, a light chain CDR2 as set forth in SEQ ID NO: 17, and a light chain CDR3 as set forth in SEQ ID NO: 18; or
the heavy chain variable region comprises a heavy chain CDR1 as set forth in SEQ ID NO: 19, a heavy chain CDR2 as set forth in SEQ ID NO: 20, and a heavy chain CDR3 as set forth in SEQ ID NO: 21; and/or the light chain variable region comprises a light chain CDR1 as set forth in SEQ ID NO: 22, a light chain CDR2 as set forth in SEQ ID NO: 23, and a light chain CDR3 as set forth in SEQ ID NO: 24; or
the heavy chain variable region comprises a heavy chain CDR1 as set forth in SEQ ID NO: 25, a heavy chain CDR2 as set forth in SEQ ID NO: 26, and a heavy chain CDR3 as set forth in SEQ ID NO: 27; and/or the light chain variable region comprises a light chain CDR1 as set forth in SEQ ID NO: 28, a light chain CDR2 as set forth in SEQ ID NO: 29, and a light chain CDR3 as set forth in SEQ ID NO: 30.

In some specific embodiments of the present invention, the antibody is a full-length antibody.

In some specific embodiments of the present invention, the antigen-binding fragment is Fab or F(ab)'2 or scFv.

In some specific embodiments of the present invention, the antibody is a chimeric antibody or a humanized antibody.

In some specific embodiments of the present invention, the heavy chain variable region comprises amino acid sequences having at least 75% identity to the sequences as set forth in SEQ ID NO: 7, SEQ ID NO: 9, or SEQ ID NO: 11.

In some specific embodiments of the present invention, the light chain variable region comprises amino acid sequences having at least 75% identity to the sequences as set forth in SEQ ID NO: 8, SEQ ID NO: 10, or SEQ ID NO: 12.

In some specific embodiments of the present invention, the heavy chain variable region comprises the sequence as set forth in SEQ ID NO: 7, and the light chain variable region comprises the sequence as set forth in SEQ ID NO: 8.

In some specific embodiments of the present invention, the heavy chain variable region comprises the sequence as set forth in SEQ ID NO: 9, and the light chain variable region comprises the sequence as set forth in SEQ ID NO: 10.

In some specific embodiments of the present invention, the heavy chain variable region comprises the sequence as set forth in SEQ ID NO: 11, and the light chain variable region comprises the sequence as set forth in SEQ ID NO: 12.

In some specific embodiments of the present invention, the heavy chain variable region comprises amino acid sequences as set forth in SEQ ID NO: 36, SEQ ID NO: 37, or SEQ ID NO: 38.

In some specific embodiments of the present invention, the light chain variable region comprises amino acid sequences as set forth in SEQ ID NO: 39 or SEQ ID NO: 40.

In some specific embodiments of the present invention, the anti-CTLA4 antibody or antigen-binding fragments thereof comprises a heavy chain constant region and/or a light chain constant region; wherein the heavy chain constant region of the antibody is a heavy chain constant region of a mouse antibody or a heavy chain constant region of a human antibody, and/or the light chain constant region of the antibody is a light chain constant region of a mouse antibody or a light chain constant region of a human antibody.

In some specific embodiments of the present invention, the anti-CTLA4 antibody or antigen-binding fragments thereof inhibits the binding of CTLA4 to human B7-1 and/or human B7-2.

In another aspect, the present invention relates to an isolated anti-CTLA4 antibody or antigen-binding fragments thereof inhibiting the binding of CTLA4 to human B7, which binds the same epitopes as any of the anti-CTLA4 antibody or antigen-binding fragments thereof above, or competes for binding human B7 with any of the anti-CTLA4 antibody or antigen-binding fragments thereof above.

In another aspect, the present invention relates to an isolated nucleic acid encoding any of the anti-CTLA4 antibody or antigen-binding fragments thereof above; a vector comprising the nucleic acid; and a host cell comprising the nucleic acid or the vector.

The chimeric antibodies provided by the present invention can bind to the activated T cells. (See Example 6)

The chimeric antibodies provided by the present invention show high affinity in the kinetic detections, and the equilibrium association constants K*_{D}* are < 1x10⁻¹² and 8.68x10⁻¹¹. In one aspect, the kinetic parameters provided by the present invention are determined using the biomolecular interaction system Octet-96 (Pall Life Sciences, S-000959). (See Example 7)

The binding epitopes of the chimeric antibodies provided by the present invention to the antigen CTLA4-hFc is not exactly same as compared to the known positive control antibodies. (See Example 8)

The chimeric antibodies provided by the present invention have higher bioactivity *in vitro.* In one aspect, antibodies of the present invention can enhance the secretion of IL-2 cytokines using the Jurkat cell detection during the cell activity experiments (see Example 9); and in another aspect, antibodies of the present invention can enhance the secretion of IFN-γ during the allogeneic mixed lymphocyte reactions (see Example 10).

The chimeric antibodies provided by the present invention have higher pharmacological activity in *vivo.* Antibodies of the present invention can effectively inhibit the tumor growth and diminish the volume of the tumor in the tumor models of animals. (See Example 11)

Antibodies of the present invention have the following uses *in vivo* and *in vitro*: block the binding of CTLA4 to B7, regulate (e.g., down-regulate) the activity or level of CTLA4, relieve the immunosuppression of CTLA4 to the organism, activate T lymphocytes, or improve the expression of IL-2 in T lymphocytes.

In another aspect, the present invention relates to an immunoconjugate comprising an antibody portion and a conjugation portion, the antibody portion is any of the anti-CTLA4 antibody or antigen-binding fragments thereof above, and the conjugation portion is a therapeutic reagent or a diagnostic reagent. Preferably, the conjugation portion is selected from one or more of a radionuclide, a drug, a toxin, a cytokine, an enzyme, a fluorescein, and a biotin.

The present invention provides a pharmaceutically effective antibody specifically binding to human CTLA4. In specific, the pharmaceutically effective human antibody binds to CTLA4 on the surfaces of T cells and other immune cells.

In another aspect, the present invention provides a pharmaceutical composition, comprising any of anti-CTLA4 antibody or antigen-binding fragments thereof as described above, the nucleic acid as described above, or the immunoconjugate as described above, and the pharmaceutically acceptable carriers. The compositions of the present invention may further comprise a conjugate of an anti-CTLA4 antibody or antigen-binding fragments thereof and one or more other bioactive molecules as separate molecules (e.g., a conjugate of at least one chimeric antibody of the present invention and another bioactive molecule), or may bind to a plurality of conjugates of one or more other bioactive molecules in one molecule, e.g., in the case of bispecific or multispecific molecules, a conjugate of two or more chimeric antibodies of the present invention or a conjugate with one or more other bioactive molecules.

In another aspect, the present invention relates to use of the anti-CTLA4 antibody or antigen-binding fragments thereof, the nucleic acid or the immunoconjugate of the present invention in the manufacture of medicaments for specifically binding CTLA4.

In some specific embodiments, medicaments specifically binding CTLA are used for blocking the binding of CTLA4 to B7, down-regulating the activity or level of CTLA4, relieving the immunosuppression of CTLA4 to an organism, activating T lymphocytes, or improving the expression of IL-2 in T lymphocytes.

In some specific embodiments, medicaments specifically binding CTLA are used for prevention and/or treatment and/or adjuvant treatment of cancers and infectious diseases.

In some specific embodiments, the cancers are selected from breast cancer, lung cancer, colorectal cancer, stomach cancer, colon cancer, esophageal cancer, ovarian cancer, cervical cancer, kidney cancer, bladder cancer, prostate cancer, pancreatic cancer, liver cancer, glioma or melanoma.

In another aspect, the present invention relates to a method of prevention and/or treatment and/or adjuvant treatment of cancers and immune system diseases, comprising administering an effective amount of pharmaceutical compositions of the present invention to subjects in need thereof.

In some specific embodiments, the cancers are selected from breast cancer, lung cancer, colorectal cancer, stomach cancer, colon cancer, esophageal cancer, ovarian cancer, cervical cancer, kidney cancer, bladder cancer, prostate cancer, pancreatic cancer, liver cancer, glioma or melanoma.

The term "identity" as used herein refers to the percentage of same amino acid residues between two or more sequences calculated using algorithms well known in the art, e.g., BLAST, with default parameters.

The term "pharmaceutically acceptable carrier" as used herein refers to solid or liquid diluents, fillers, antioxidants, stabilizers and the like which can be administrated safely. According to the routes of administration, A variety of different carriers well known in the art can be administrated, including but not limited to sugars, starches, cellulose and the derivatives thereof, maltose, gelatin, talc, calcium sulfate, vegetable oils, synthetic oils, polyols, alginate, phosphoric acid buffer, emulsifiers, isotonic saline, and/or pyrogen-free water, etc.

The term "effective amount" as used herein refers to the amount of active compound that is sufficient to lead to biologic or medical responses as desired by clinicians in the subjects. The "therapeutically effective amount" of the anti-CTLA4 antibody or antigen-binding fragments thereof can be determined by one skilled in the art according to factors, such as the routes of administration, and patients' weight, age, illness. For example, the typical daily dose may range from 0.01 mg to 100 mg of active ingredient per kg of body weight.

The medicine provided by the present invention can be made into clinically acceptable dosage forms such as powder, injection and the like. The pharmaceutical compositions of the present invention may be administered to a subject using any suitable routes, e.g., it may be administrated orally, by intravenous infusion, by intramuscular injection, by hypodermic injection, subperitoneally, rectally, sublingually, or by inhalation, transdermally, etc.

As used herein, the term "comprise" typically means including but not limited to. When referring to "comprise" a certain sequence, it also should be considered to comprise cases consisting of this sequence.

Those skilled in the art will understand that in addition to those described herein, antibodies of the present invention may have additional uses or compositions. In all cases where the antibody binds to human CTLA4 antigen, such uses and compositions are considered to be within the scope of the present invention. Those skilled in the art will understand that changes may be made to variable region sequences of the antibodies of the present invention (SEQ ID NO: 7 to SEQ ID NO: 12), or CDRs of the antibodies of the present invention (SEQ ID NO: 13 to SEQ ID NO: 30) or sequences of the humanized antibodies (SEQ ID NO: 31 to SEQ ID NO: 45). The changes will not significantly change the properties of antibodies of the present invention, and such variants thus should be considered to be within the scope of the present invention. Additionally, such changes within the variable regions or CDR sequences of the antibodies are considered to be within the scope of the present invention so long as the variable region sequences of such variants have significant homology to the sequences of the present invention.

### BRIEF DESCRIPTION OF THE DRAWINGS

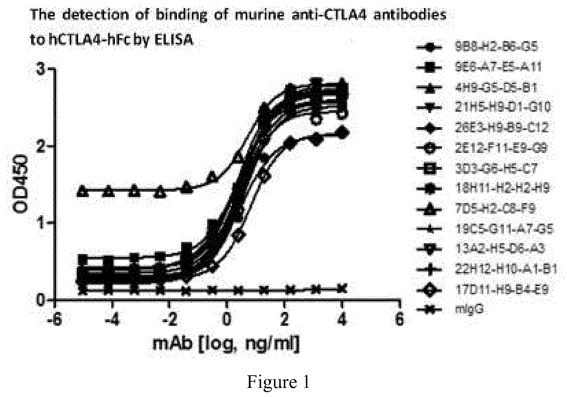

Fig.1: The detection of binding of murine anti-CTLA4 antibodies to hCTLA4-Fc protein by ELISA.

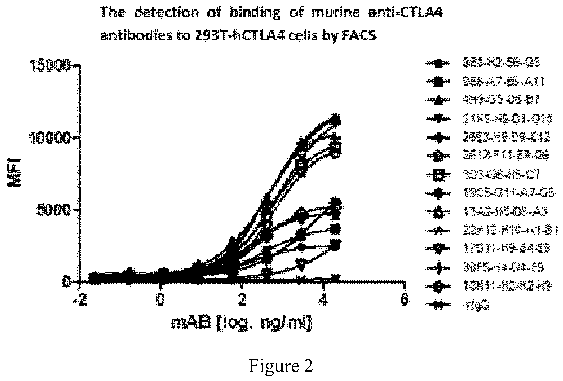

Fig.2: The detection of binding of murine anti-CTLA4 antibodies to 293T-hCTLA4 cells by FACS.

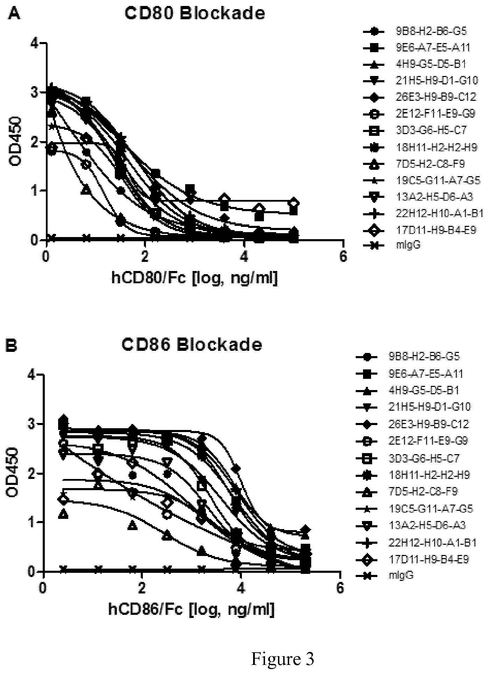

Fig.3: The detection of murine anti-CTLA4 antibodies blocking the binding of CD80 or CD86 to CTLA4 by ELISA.

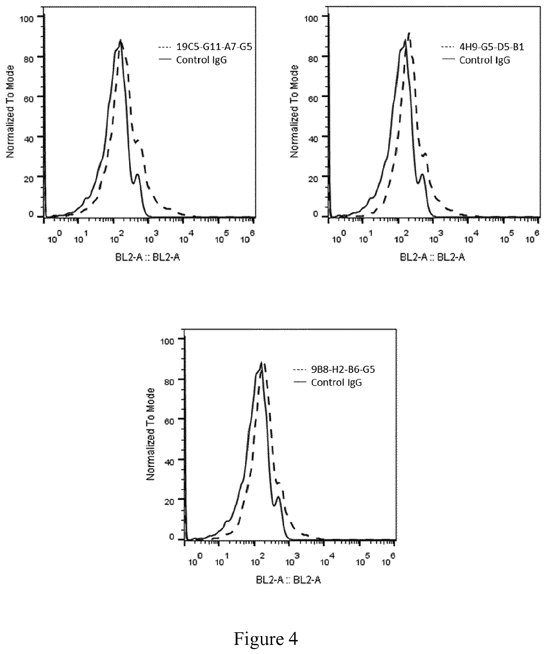

Fig.4: The detection of binding of CTLA4 chimeric antibodies to the activated T cells by FACS.

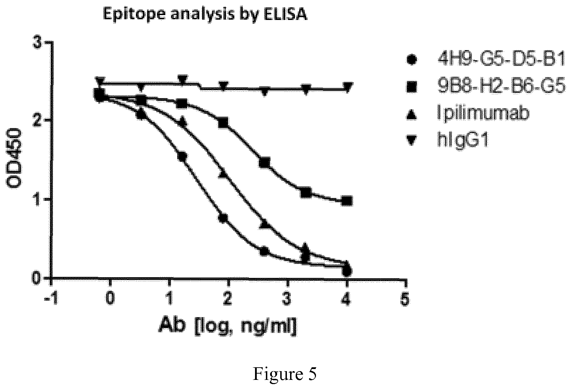

Fig.5: The detection of epitope competition between the CTLA4 chimeric antibodies and other antibodies by ELISA.

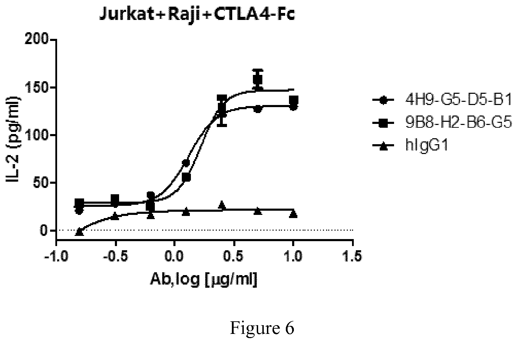

Fig.6: The detection of CTLA4 chimeric antibodies dose-dependently enhancing IL-2 secretion in Jurkat cells by ELISA.

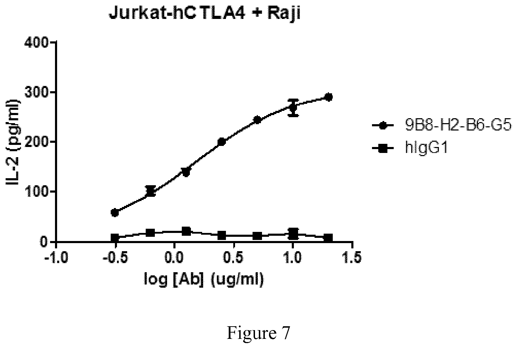

Fig.7: The detection of CTLA4 chimeric antibodies dose-dependently enhancing IL-2 secretion in Jurkat-hCTLA4 cells by ELISA.

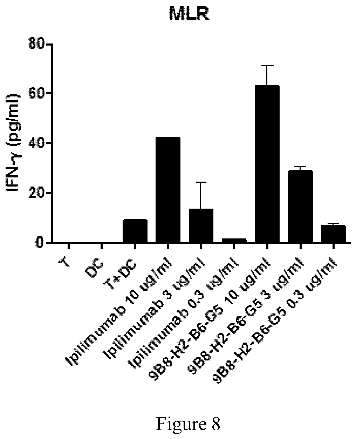

Fig.8: The detection of CTLA4 chimeric antibodies enhancing IFN-γ secretion in MLR experiments by ELISA.

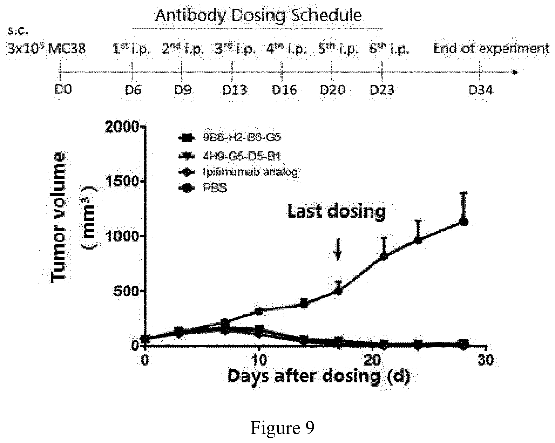

Fig.9: Tumor growth inhibition in the human CTLA4 knock-in mice by CTLA4 chimeric antibodies.

### DETAILED DESCRIPTION OF THE INVENTION

The embodiments of the present invention will hereinafter be described in detail with reference to Examples. Those skilled in the art will understand that the Examples below are only intended to illustrate the invention, but not to limit the scope of the invention. Examples in which specific techniques or conditions are not indicated are carried out according to the techniques or conditions described in the literatures in the art (e.g., with reference to "Molecular Cloning: A Laboratory Manual", Sambrook. J. et al. (authors), Huang Peitang et.al. (translators), the 3rd edition, Science Press) or according to the product specifications. Reagents or instruments in which the manufacturers are not indicated are conventional products that are commercially available.

### Example 1: Animal immunization and screening of mouse anti-CTLA4 antibodies

Age-appropriate Balb/c mice were selected and subjected immune injection. The hCTLA4-mFc fusion protein was used as an antigen and mixed with complete Freund's adjuvant (Sigma-Aldrich), and then injected into the immunized mice by subcutaneous injection to stimulate the cloning of corresponding B-lymphocytes. Then the immunized mice were boosted by the intraperitoneal injection of 100 µg of CTLA4-mFc emulsified with incomplete Freund's adjuvant (Sigma-Aldrich) at a ratio of 1:1 about every two to three weeks. Spleen lymphocytes taken from the mice by sterile operations were mixed with the prepared SP2/0 myeloma cells in a certain ratio (1x10⁸ of spleen cells and 2x10⁷ of myeloma cells) with the addition of polyethylene glycol (Sigma, P7181).

After fusion, the fused cells were added into 96-well plates with 0.1 mL of HAT medium per well and were cultured in the CO₂ incubator at 37°C. On day 4, 0.1 mL of HT medium per well was added. On day 7, the culture medium was completely replaced with HT medium. The screening was carried out at days 8-12. The positive cells were sub-cloned by limiting dilution, and were expanded and tested by ELISA and FACS as described below.

The 2 µg/mL of hCTLA4-hFc was coated on the 96-well plates (Corning, catalog #9018) followed by overnight incubation at 4°C. The plates were washed with PBST (PBS containing 0.05% of Tween-20) three times, and were blocked with 5% skimmed milk powder (Oxoid, catalog #LP0031B). After washing the plates with PBST three times, the hybridoma supernatants were added and incubated for 2 hours at 37°C. The plates were washed and incubated for 1 hour at 37°C with 1/5000 diluted HRP-labeled goat anti-mouse IgG second antibodies (BioLegend, catalog #405306). The plates were washed again, and were developed by adding TMB (Tiangen catalog #PA107-02).

The binding of antibodies to 293T-hCTLA4 cells was detected by FACS. 1x10⁵ of 293T-hCTLA4 cells per well were added into 96-well plates (Corning, catalog #3799) and were incubated with the hybridoma supernatants for 30 minutes at 4°C. After the cells were washed twice with buffer (PBS containing 1% BSA and 5 mM EDTA), the PE-labeled goat anti-mouse IgG secondary antibodies were added and incubated for 30 minutes at 4°C. The cells were washed with buffer and analyzed by Attune Nxt flow cytometer (Thermo Fisher).

The hybridoma supernatants were further screened by employing ligand blocking experiments, since CTLA4 antibodies can block the binding of CTLA4 to CD80. The 2 µg/mL goat anti-mouse IgG antibodies (Sigma, catalog #M4280) were coated on the 96-well plates (Corning, catalog #9018) followed by overnight incubation at 4°C. The plates were washed three times and blocked with 5% skimmed milk powder (Oxoid, catalog #LP0031B) for 2 hours at 37°C. The plates were washed three times and were incubated for 1 hour at room temperature with the pre-mixture of 20 µg/mL hCD80-Fc and 20 ng/mL biotinylated hCTLA4-His. The plates were washed and incubated with HRP-labeled streptavidin (BD, catalog #554066) for 1 hour at room temperature. The plates were washed and developed by adding TMB.

### Example 2: Physicochemical and binding properties of purified hybridoma supernatants anti-CTLA4 antibodies

Monoclonal cells of the hybridomas were obtained by three or more rounds of limiting dilution. Stable monoclonal cells of the hybridomas were carried out the serum-free domestication (Gibco, catalog # 12045-076) for 8 to 9 days and were expanded to T-175 culture flask (NUNC, catalog #159910). On days 8-9, the supernatant was collected and filtered using a 0.22 µm filter membrane. The supernatant was passed through the Protein A column (GE Healthcare, catalog #17549112) to isolate and purify the antibodies, which were adsorbed on the column. The Protein A column was washed with 1 mM PBS, and the antibodies were eluted from the column using 50 mM PBS (pH3.0). The eluate was adjusted to neutrality with a 0.5 M of sodium hydroxide solution and filtered using a 0.22 µm filter membrane. The solution of antibodies was concentrated by Ultra-15 centrifugal concentrators (Millipore, catalog #ACS500024), and the concentration of the antibodies was detected using Nanodrop spectrophotometry (Thermo Scientific, NanoDrop 2000). The content of endotoxin in the solution of purified antibodies was detected using the Gel Clot TAL kit (Xiamen Bioendo Technology, catalog #010250) with a standard content of less than 1 EU/mL.

ELISA was used for detecting the binding of purified antibodies to CTLA4-hFc. The hCTLA4-hFc was coated on 96-well plates (Corning, catalog #9018) followed by incubation overnight at 4°C. The plates were washed with PBST (PBS containing 0.05% of Tween-20) three times, and were blocked with 5% skimmed milk powder (Oxoid, catalog #LP0031B) for 2 hours at 37°C. The plates were washed three times with PBST and were incubated for 1 hour at room temperature with antibodies to be detected. The plates were washed three times and incubated for 1 hour at room temperature with 1/5000 diluted HRP-labeled goat anti-mouse IgG secondary antibodies (Invitrogen, catalog #31432). The plates were washed with PBST and were developed by adding TMB. The results are shown in FIG.1, and the median effective concentrations of binding are shown in Table I.

The purified antibodies were detected for the binding to 293T-hCTLA4 cells by employing FACS. 293T-hCTLA4 cells were resuspended to 4x10⁶ cells/mL with FACS buffer (PBS containing 1% BSA) and added to the 96-well round-bottom plates (Corning, catalog #3795) at 50 µL/well. The purified antibodies to be detected were added to wells at a certain concentration (50 µL/well) and incubated for 1 hour at 4°C. The cells were washed three times with buffer and were incubated for 0.5 hour at 4°C with PE-labeled fluorescent secondary antibodies. The cells were washed three times with FACS buffer and re-suspended to 200 µL/well. The fluorescence signals were detected using Attune Nxt flow cytometer (Thermo Fisher). The results are shown in FIG. 2, and the median effective concentrations of binding are shown in Table I.

**Table I**

| binding force analysis of murine anti-CTLA4 monoclonal antibody | | |
|---|---|---|
| Antibody ID | ELISA EC₅₀ (nM) | FACS EC₅₀ (nM) |
| | hCTLA4-hFc | 293T-hCTLA4 |
| 9B8-H2-B6-G5 | 0.025 | 1.08 |
| 9E6-A7-E5-A11 | 0.023 | 2.28 |
| 4H9-G5-D5-B1 | 0.023 | 1.35 |
| 21H5-H9-D1-G10 | 0.026 | 5.52 |
| 26E3-H9-B9-C12 | 0.022 | 1.15 |
| 2EI2-F11-E9-G9 | 0.028 | 4.11 |
| 3D3-G6-H5-C7 | 0.029 | 3.49 |
| 7D5-H2-C8-F9 | 0.047 | |
| 13A2-H5-D6-A3 | 0.032 | 3.56 |
| 17D11-H9-B4-E9 | 0.059 | |
| 18H11-H2-H2-H9 | 0.021 | 2.02 |
| 19C5-G11-A7-G5 | 0.036 | |
| 22H12-H10-A1-B1 | 0.025 | 3.27 |
| 30F5-H4-G4-F9 | | 2.34 |

### Example 3: Blocking of interactions between CD80 or CD86 and CTLA4 by murine CTLA4 antibody

ELISA was used to detect whether the antibody blocked the binding between CD80 or CD86 and CTLA4. The hCTLA4-hFc protein was labeled with biotin using the Biotin Labeling Kit-NH₂ (Dojindo, catalog#LK03). The purified antibodies were coated on 96-well plates (Corning, catalog #9018) followed by incubation overnight at 4°C, and were blocked with 5% skimmed milk powder (Oxoid, catalog #LP0031B) for 2 hours at 37°C. The plates were washed three times, and incubated for 1 hour at room temperature with the pre-mixture of biotinylated hCTLA4-hFc and hCD80-Fc or hCD86-Fc. The plates were washed with PBST and incubated with HRP-labeled streptavidin (BD, catalog #554066) for 1 hour at room temperature. The plates were washed and developed by adding TMB. The results are shown in FIG.3, and the results are summarized in Table II.

**Table II**

| ligand blocking experiments of murine anti-CTLA4 antibodies | | |
|---|---|---|
| | ELISA IC₅₀ (ng/mL) | |
| Antibody ID | CD80 | CD86 |
| 9B8-H2-B6-G5 | 2.917 | 1079 |
| 9E6-A7-E5-A11 | 32.58 | 5824 |
| 4H9-G5-D5-B1 | 69.06 | 7244 |
| 21H5-H9-D1-G10 | 26.27 | 4089 |
| 26E3-H9-B9-C12 | 52.26 | 10058 |
| 2E12-F11-E9-G9 | 14.41 | |
| 3D3-G6-H5-C7 | 23.62 | 2442 |
| 7D5-H2-C8-F9 | | 272.5 |
| 13A2-H5-D6-A3 | 29.5 | 2091 |
| 17D11-H9-B4-E9 | 44.46 | 2286 |
| 18H11-H2-H2-H9 | 46.73 | 7293 |
| 19C5-G11-A7-G5 | 35.69 | 3145 |
| 22H12-H10-A1-B1 | 62.36 | 8395 |

### Example 4: Species cross reactions of murine CTLA4 antibodies

Species cross reactions of the purified antibodies were detected using ELISA. The quantified purified antibodies were coated on 96-well plates (Corning, catalog #9018) followed by incubation overnight at 4°C, and were blocked with 5% skimmed milk powder (Oxoid, catalog #LP0031B) for 2 hours at 37°C. The biotin-labeled mouse CTLA4-His (SinoBiological, catalog #50503-M08H) or monkey CTLA4-Fc (SinoBiological, catalog #90213-C02H) was added to the plates and incubated for 1 hour at room temperature. The plates were washed three times with PBST and incubated with 1/1000 diluted HRP-labeled streptavidin (BD, catalog #554066) for 1 hour at room temperature. The plates were washed and developed by adding TMB. The results are shown in Table III.

**Table III**

| cross reactions of murine anti-CTLA4 antibodies | | |
|---|---|---|
| | ELISA (OD) | |
| Antibody ID | mouse | monkey |
| 9B8-H2-B6-G5 | 0.06 | 0.37 |
| 9E6-A7-E5-A11 | 0.05 | 0.87 |
| 4H9-G5-D5-B1 | 0.05 | 2.79 |
| 21H5-H9-D1-G10 | 0.06 | 2.06 |
| 26E3-H9-B9-C12 | 0.06 | 1.83 |
| 2EI2-F11-E9-G9 | 0.05 | 0.58 |
| 3D3-G6-H5-C7 | 0.06 | 2.42 |
| 13A2-H5-D6-A3 | 0.05 | 2.36 |
| 17D11-H9-B4-E9 | 0.05 | 0.62 |
| 18H11-H2-H2-H9 | 0.06 | 2.93 |
| 19C5-G11-A7-G5 | 0.06 | 0.64 |
| 22H12-H10-A1-B1 | 0.05 | 2.91 |
| 30F5-H4-G4-F9 | 0.05 | 2.64 |

### Example 5: Cloning of cDNAs of murine antibodies and construction of chimeric antibodies

Using degenerate primer PCR-based methods, the gene sequences of variable regions of the heavy and light chains of the hybridoma antibodies are obtained. Monoclonal cells of hybridoma were lysed using Trizol (Invitrogen, catalog #15596-018) to isolate total RNA, and reverse transcription was performed using RNA as a template with SuperScript III First-Strand Synthesis System (Invitrogen, catalog #18080-051) to obtain a cDNA bank. The resulting cDNA bank was used as a template, and PCR was performed using degenerate primers (Zhou H, et al., Nucleic Acids Research 22: 888-889 (1994), Chardes T et al., FEBS Letters 452: 386-394 (1999)). PCR products were detected by agarose gel electrophoresis. The size of the PCR amplification product of the heavy and light chain variable regions is expected to be 400 base pairs. The PCR products were cloned to the pClone007 vector (Tsingke, catalog #TSV-007BS) and transformed to Trans 5α, a competence of Escherichia coli, (Transgen, catalog# CD201-02). 3-6 monoclonal Escherichia colis were selected on agar plates for gene sequencing. In some cases, PCR products may also be directly used for gene sequencing. The full-length gene sequences of the variable regions of the heavy and light chains of antibodies 4H9-G5-D5-B1, 9B8-H2-B6-G5, and 19C5-G11-A7-G5 were finally obtained through the methods above. The gene and amino acid sequences of the complementarity determining regions and framework regions of the heavy and light chains of the antibodies were further obtained through analysis using NCBI Ig-BLAST (https://www.ncbi.nlm.nih.gov/projects/igblast/) (Kabat E.A., et al., 1991, Sequences of proteins of immunological interest, in: NIH Publication No. 91-3242, US Department of Health and Human Services, Bethesda, MD). The detailed information was disclosed in the sequencing listing of antibodies and Table IV.

The light and heavy chains of chimeric antibody were constructed by linking the variable regions of murine CTLA4 antibody to human IgG1 and kappa constant regions. The appropriate restriction sites were introduced into the variable regions of the heavy and light chains by PCR, and cloned into the expression vectors of corresponding chimeric antibodies, respectively. The plasmids of heavy and light chains of chimeric antibodies were introduced into 293F cells by lipofection and cultured for 6 days. The antibodies in the cell culture supernatant were purified with Protein A column (GE Healthcare, catalog #17549112). The Protein A column was washed with 1 mM PBS, and the antibodies were eluted from the column using 50 mM of PBS (pH3.0). The eluate was adjusted to neutrality with a 0.5 M of sodium hydroxide solution and filtered using a 0.22 µm filter membrane. The solution of antibodies was concentrated by Ultra-15 centrifugal concentrators (Millipore, catalog #ACS500024), and the concentration of the antibodies was detected using Nanodrop spectrophotometry. The content of endotoxin in the solution of purified antibodies was detected using the Gel Clot TAL kit (Xiamen Bioendo Technology, catalog #010250) with a standard content of less than 1 EU/mL. The binding activity of the chimeric antibodies produced by the transfected 293F cells was detected by ELISA and FACS (see Example 2 for specific steps). The results showed that chimeric antibodies bind to CTLA4 with comparable affinity to those of the murine antibodies.

**Table IV**

| Sequence ID number of anti-CTLA4 antibodies in this invention | |
|---|---|
| SEQ ID NO: | Description |
| 1 | 9B8-H2-B6-G5 heavy chain variable region (DNA) |
| 2 | 9B8-H2-B6-G5 light chain variable region (DNA) |
| 3 | 4H9-G5-D5-B1 heavy chain variable region (DNA) |
| 4 | 4H9-G5-D5-B1 light chain variable region (DNA) |
| 5 | 19C5-G11-A7-G5 heavy chain variable region (DNA) |
| 6 | 19C5-G11-A7-G5 light chain variable region (DNA) |
| 7 | 9B8-H2-B6-G5 heavy chain variable region (AA) |
| 8 | 9B8-H2-B6-G5 light chain variable region (AA) |
| 9 | 4H9-G5-D5-B1 heavy chain variable region (AA) |
| 10 | 4H9-G5-D5-B1 light chain variable region (AA) |
| 11 | 19C5-G11-A7-G5 heavy chain variable region (AA) |
| 12 | 19C5-G11-A7-G5 light chain variable region (AA) |
| 13 | 9B8-H2-B6-G5 heavy chain CDR1 (AA) |
| 14 | 9B8-H2-B6-G5 heavy chain CDR2 (AA) |
| 15 | 9B8-H2-B6-G5 heavy chain CDR3 (AA) |
| 16 | 9B8-H2-B6-G5 light chain CDR1 (AA) |
| 17 | 9B8-H2-B6-G5 light chain CDR2 (AA) |
| 18 | 9B8-H2-B6-G5 light chain CDR3 (AA) |
| 19 | 4H9-G5-D5-B1 heavy chain CDR1 (AA) |
| 20 | 4H9-G5-D5-B1 heavy chain CDR2 (AA) |
| 21 | 4H9-G5-D5-B1 heavy chain CDR3 (AA) |
| 22 | 4H9-G5-D5-B1 light chain CDR1 (AA) |
| 23 | 4H9-G5-D5-B1 light chain CDR2 (AA) |
| 24 | 4H9-G5-D5-B1 light chain CDR3 (AA) |
| 25 | 19C5-G11-A7-G5 heavy chain CDR1 (AA) |
| 26 | 19C5-G11-A7-G5 heavy chain CDR2 (AA) |
| 27 | 19C5-G11-A7-G5 heavy chain CDR3 (AA) |
| 28 | 19C5-G11-A7-G5 light chain CDR1 (AA) |
| 29 | 19C5-G11-A7-G5 light chain CDR2 (AA) |
| 30 | 19C5-G11-A7-G5 light chain CDR3 (AA) |
| 31 | 9B8-VH4 heavy chain variable region (DNA) |
| 32 | 9B8-VH5 heavy chain variable region (DNA) |
| 33 | 9B8-VH6 heavy chain variable region (DNA) |
| 34 | 9B8-VL4 light chain variable region (DNA) |
| 35 | 9B8-VL5 light chain variable region (DNA) |
| 36 | 9B8-VH4 heavy chain variable region (AA) |
| 37 | 9B8-VH5 heavy chain variable region (AA) |
| 38 | 9B8-VH6 heavy chain variable region (AA) |
| 39 | 9B8-VL4 light chain variable region (AA) |
| 40 | 9B8-VL5 light chain variable region (AA) |
| 41 | 9B8-VH4 full length heavy chain (AA) |
| 42 | 9B8-VH5 full length heavy chain (AA) |
| 43 | 9B8-VH6 full length heavy chain (AA) |
| 44 | 9B8-VL4 full length light chain (AA) |
| 45 | 9B8-VL5 full length light chain (AA) |

### Example 6: Binding of human-mouse CTLA4 chimeric antibodies to activated human T cells

Binding of the CTLA4 chimeric antibodies to activated human T cells was detected by FACS. Human peripheral blood mononuclear cells (PBMCs) were isolated from human peripheral blood using density gradient centrifugation (Ficoll-Paque Premium, GE Healthcare, catalog #17-5442-02). T cells were further purified from PBMC using the Pan T Isolation Kit (Miltenyi, catalog #130-096-535). T cells were resuspended in the medium (RPMI-1640 medium (Gibco, catalog #C22400500BT), 10% inactivated FBS (Gibco, catalog #10099-141) and penicillin/penicillin diabody (Gibco, catalog #15140-122)), and were activated using Dynabeads® Human T-Activator CD3/CD28 (Gibco, catalog #11132D) according to instructions. After 3 days, the activated T cells were incubated with 10 µg/mL of chimeric antibodies in buffer (PBS containing 1% BSA) for 1 hour at 4°C. The cells were washed three times and incubated with the PE-labeled mouse anti-human IgG Fc secondary antibodies (BioLegend, catalog #409304) for 1 hour at 4°C. The T cells were washed three times with buffer and resuspended in 200 µL/well buffer prior to detecting the fluorescence signal on Attune Nxt flow cytometer (Thermo Fisher). The results are shown in FIG. 4.

### Example 7: Kinetic detections of human-mouse CTLA4 chimeric antibodies

The kinetic constants (k*_{assoc}* and k*_{dissoc}*) of binding of the antibody and the antigen were measured using the biomolecular interaction system Octet-96 (Pall Life Sciences, S-000959), and the equilibrium binding constant K*_{D}* was further calculated. The CTLA4-mFc antigen proteins were coupled to the surfaces of an AMC sensors (Pall Life Sciences, PN18-5099), and antibodies of different concentrations were added to measure the binding and dissociation between the CTLA4 chimeric antibodies and the CTLA4-mFc proteins on the sensor surfaces. Specifically, AMC sensors were pre-wet in the buffer (PBS containing 0.02% Tween-20 and 0.1% BSA) for 10 minutes and then equilibrated in CTLA4-mFc sample buffer for 5 minutes to such that the CTLA4-mFc proteins were coupled to the surfaces of sensors. The CTLA4-mFc-coupled AMC sensors were first equilibrated in the buffer for 2 minutes and then incubated for 5 minutes in buffer containing different concentrations of antibody (3-200 nM) to measure the binding of antibodies to the CTLA4-mFc protein. Finally, the antigen and antibody-bound sensors were repositioned in the sample buffer and waited for 10 minutes to measure the dissociation of antibodies from the CTLA4-mFc proteins. The measured data were globally fitted using a 1:1 model to obtain kinetic constants of binding and dissociation. On this basis, an equilibrium binding constant K*_{D}* was further obtained. Experimental results are presented in Table V.

**Table V**

| Kinetic Analysis (ForteBio) | | | |
|---|---|---|---|
| | k*_{assoc}* (1/s) | k*_{dissoc}* (1/Ms) | K*_{D}* (M) |
| 4H9-G5-D5-B1 | 1.10E+06 | <1.0E-07 | <1.0E-12 |
| 9B8-H2-B6-G5 | 4.78E+05 | 4.14E-05 | 8.68E-11 |

### Example 8: Epitope analysis of human-mouse CTLA4 chimeric antibodies

ELISA was used to detect the epitope competition between the CTLA4 chimeric antibodies and reference antibodies. The 2 µg/mL of Ipilimumab was coated on the 96-well plates (Corning, catalog #9018) followed by incubation overnight at 4°C, blocked with 5% skimmed milk powder (Oxoid, catalog #LP0031B) for 2 hours at 37°C, and washed three times with PBST. The biotin-labeled CTLA4-hFc were pre-incubated with gradient-diluted chimeric antibodies for 30 minutes at room temperature, added to the 96-well plates and incubated for 1 hour at room temperature. The plates were washed three times with PBST and incubated with 1/1000 diluted HRP-labeled streptavidin (BD, catalog #554066) for 1 hour at room temperature. The plates were washed and developed by adding TMB. The results are shown in FIG.5. The binding of Ipilimumab to CTLA4-hFc can be completely blocked by chimeric antibody 4H9-G5-D5-B1. In contrast, the binding of Ipilimumab to CTLA4-hFc cannot be completely blocked by chimeric antibody 9B8-H2-B6-G5, indicating that these two antibodies have different binding epitopes to antigen CTLA4-hFc.

### Example 9: Enhancement of immune function of Jurkat cells by human-mouse CTLA4 chimeric antibodies

Jurkat cells were used to detect the cellular biological activity of human-mouse CTLA4 chimeric antibodies. One experiment used Raji cells as the antigen presenting cells to stimulate Jurkat cells, which could be blocked by adding exogenous CTLA4-hFc protein. The addition of CTLA4 antibody in the systems above could neutralize the exogenous CTLA4-hFc protein and recover the function of Jurakt cells to activate Jurkat cells. Specifically, Raji cells were treated with 25 µg/mL mitomycin C (MedChem Express, catalog #HY-13316) for 1 hour at 37°C, and were washed 4 times with PBS. Jurkat cells and treated Raji cells were resuspended in medium (RPMI-1640 with 10% inactivated FBS) and added to 96-well round-bottom plates (Corning, catalog #3799) with an equal amount of anti-CD3 antibody (BioLengend, catalog #317326), CTLA4-hFc fusion protein, as well as test antibodies at varying concentrations. The supernatants were collected after 2 days of culture of cells, and the content of IL-2 in supernatants was quantified using ELISA (antibodies were purchased from BD). The results showed that CTLA4 antibodies could enhance the secretion of IL-2 and showed a dose-dependent effect as shown in FIG. 6.

In another experiment, a Jurkat-hCTLA4 cell line was used to evaluate the in vitro biological activity of anti-CTLA4 antibodies. The hCTLA4 plasmid was introduced into Jurkat cells by electroporation (Lonza), and then cloned and screened with hygromycin to obtain a Jurkat-hCTLA4 stable cell line. Jurkat-hCTLA4 cells and mitomycin C-treated Raji cells were added into 96-well round-bottom plates with CTLA4 antibodies at varying concentrations. The supernatants were collected after 2 days of culture of cells, and IL-2 was quantitatively detected using ELISA as mentioned above. The results showed that CTLA4 antibodies could enhance the secretion of IL-2 and showed a dose-dependent effect as shown in FIG. 7.

### Example 10: Allogeneic Mixed Lymphocyte Reaction (MLR) of human-mouse CTLA4 chimeric antibodies

Mixed lymphocyte reaction was used to detect the in vitro biological activity of CTLA4 antibodies. Peripheral blood monocytes (PBMCs) were isolated from human peripheral blood using density gradient centrifugation (Ficoll-Paque Premium, GE Healthcare, catalog #17-5442-02). Monocytes were further purified from PBMCs using CD14 Cell Isolation Kit (Miltenyi, catalog #130-050-201). Cytokines 1000 U/mL GM-CSF (Prospec, catalog #CYT-221) and 1000 U/mL IL-4 (Prospec, catalog #CYT211) were added to the medium to induce monocyte differentiation into dendritic cells. Cytokines were supplemented every 2-3 days, and immature dendritic cells were harvested after 5-6 days for subsequent experiments.

Human CD3⁺ T cells were isolated from PBMCs using Pan T Cell Isolation Kit (Miltenyi, catalog #130-096-535). The CD3⁺ T cells, immature dendritic cells and CTLA4 antibodies of various concentrations were added to 96-well round-bottom plates (Corning, catalog #3799). After 5-6 days of cell culture, the supernatants were harvested, and the content of cytokines was quantitatively detected using ELISA. The results were shown in FIG. 8 that human-mouse CTLA4 chimeric antibodies could enhance the secretion of IFN-γ in MLR.

### Example 11: Tumor growth inhibition of human-mouse CTLA4 chimeric antibodies

The tumor animal model in the present invention was a special tumor animal model, which was constructed by inoculating MC38 murine colon cancer cells (NCI) in CTLA4 humanized transgenic mice (developed by Nanjing Galaxy Biopharm Co, Ltd) to test the druggability and the *in vivo* efficacy of CTLA4 antibodies in mice.

CTLA4 humanized transgenic mice (7-11 weeks old) were injected subcutaneously in the flank with 3×10⁵ MC38 tumor cells in 0.1 mL volume. After 6 days, when the tumor volume reached 70 mm³, the CTLA4 antibody, reference antibody and control PBS were injected biweekly at a dose of 10 mg/kg (dosing volume 10 mL/kg) for a total of 6 doses. The volume of subcutaneous tumor was measured biweekly using vernier calipers during the course of the experiment. Animals were followed until day 34 after injection of tumor cells. As shown in FIG.9, after the 6 doses, the tumor growth is significantly inhibited by the antibody 9B8-H2-B6-G5 to be detected, and some tumor is nearly disappeared after the treatment of drug.

### Example 12: Humanization of human-mouse chimeric CTLA4 antibodies

Murine antibody 9B8-H2-B6-G5 was humanized by CDR Grafting techniques. According to the literature reports, framework regions of subtype III of heavy chain and subtype I of kappa light chains in human antibodies have been widely used in antibody humanization (Carter P et al., PNAS 89: 4285-4289 (1992); Presta LG et al., Cancer Research 57: 4593-4599 (1997)). The CDR regions of murine antibody 9B8-H2-B6-G5 were grafted onto the framework regions of human antibodies above, and the amino acid residues in the framework regions were mutated to enhance the affinity of the antibodies. The affinity of the resultant humanized antibody for CTLA4-mFc was detected using Octet system and the AMC sensor so as to screen the best one. DNA and amino acid sequences of the heavy chains and the light chains (9B8-VH4, 9B8-VH5, 9B8-VH6, 9B8-VL4, 9B8-VL5) of the humanized antibody are shown in table IV.

Although the present invention has been described with reference to specific embodiments, those skilled in the art will recognize that, according to all the disclosed teachings, various changes and replacements may be made thereto within the scope of the present invention as set forth in the appended claims and any equivalent thereof.

## Claims

1. An isolated anti-CTLA4 antibody or antigen-binding fragments thereof inhibiting the binding of CTLA4 to human B7, the anti-CTLA4 antibody or antigen-binding fragments thereof comprises heavy and light chain variable regions, wherein:
the heavy chain variable region comprises a heavy chain CDR1 as set forth in SEQ ID NO: 13, a heavy chain CDR2 as set forth in SEQ ID NO: 14, and a heavy chain CDR3 as set forth in SEQ ID NO: 15; and/or the light chain variable region comprises a light chain CDR1 as set forth in SEQ ID NO: 16, a light chain CDR2 as set forth in SEQ ID NO: 17, and a light chain CDR3 as set forth in SEQ ID NO: 18; or
the heavy chain variable region comprises a heavy chain CDR1 as set forth in SEQ ID NO: 19, a heavy chain CDR2 as set forth in SEQ ID NO: 20, and a heavy chain CDR3 as set forth in SEQ ID NO: 21; and/or the light chain variable region comprises a light chain CDR1 as set forth in SEQ ID NO: 22, a light chain CDR2 as set forth in SEQ ID NO: 23, and a light chain CDR3 as set forth in SEQ ID NO: 24; or
the heavy chain variable region comprises a heavy chain CDR1 as set forth in SEQ ID NO: 25, a heavy chain CDR2 as set forth in SEQ ID NO: 26, and a heavy chain CDR3 as set forth in SEQ ID NO: 27; and/or the light chain variable region comprises a light chain CDR1 as set forth in SEQ ID NO: 28, a light chain CDR2 as set forth in SEQ ID NO: 29, and a light chain CDR3 as set forth in SEQ ID NO: 30.

2. The anti-CTLA4 antibody or antigen-binding fragments thereof of claim 1, the antibody is a full-length antibody.

3. The anti-CTLA4 antibody or antigen-binding fragments thereof of claim 1, the antigen-binding fragment is Fab or F(ab)'2 or scFv.

4. The anti-CTLA4 antibody or antigen-binding fragments thereof of any one of claims 1-3, the antibody is a chimeric antibody or a humanized antibody.

5. The anti-CTLA4 antibody or antigen-binding fragments thereof of any one of claims 1-4, wherein the heavy chain variable region comprises amino acid sequences having at least 75% identity to the sequences as set forth in SEQ ID NO: 7, SEQ ID NO: 9, or SEQ ID NO: 11.

6. The anti-CTLA4 antibody or antigen-binding fragments thereof of any one of claims 1-5, wherein the light chain variable region comprises amino acid sequences having at least 75% identity to the sequences as set forth in SEQ ID NO: 8, SEQ ID NO: 10, or SEQ ID NO: 12.

7. The anti-CTLA4 antibody or antigen-binding fragments thereof of claim 6, wherein the heavy chain variable region comprises the sequence as set forth in SEQ ID NO: 7, and the light chain variable region comprises the sequence as set forth in SEQ ID NO: 8.

8. The anti-CTLA4 antibody or antigen-binding fragments thereof of claim 6, wherein the heavy chain variable region comprises the sequence as set forth in SEQ ID NO: 9, and the light chain variable region comprises the sequence as set forth in SEQ ID NO: 10.

9. The anti-CTLA4 antibody or antigen-binding fragments thereof of claim 6, wherein the heavy chain variable region comprises the sequence as set forth in SEQ ID NO: 11, and the light chain variable region comprises the sequence as set forth in SEQ ID NO: 12.

10. The anti-CTLA4 antibody or antigen-binding fragments thereof of any one of claims 1-6, wherein the heavy chain variable region comprises amino acid sequences as set forth in SEQ ID NO: 36, SEQ ID NO: 37, or SEQ ID NO: 38.

11. The anti-CTLA4 antibody or antigen-binding fragments thereof of any one of claims 1-6 or 10, wherein the light chain variable region comprises amino acid sequences as set forth in SEQ ID NO: 39 or SEQ ID NO: 40.

12. The anti-CTLA4 antibody or antigen-binding fragments thereof of any one of claims 1-11, the anti-CTLA4 antibody or antigen-binding fragments thereof comprises a heavy chain constant region and/or a light chain constant region; wherein the heavy chain constant region of the antibody is a heavy chain constant region of a mouse antibody or a heavy chain constant region of a human antibody, and/or the light chain constant region of the antibody is a light chain constant region of a mouse antibody or a light chain constant region of a human antibody.

13. The anti-CTLA4 antibody or antigen-binding fragments thereof of any one of claims 1-12, the anti-CTLA4 antibody or antigen-binding fragments thereof inhibits the binding of CTLA4 to human B7-1 and/or human B7-2.

14. An isolated anti-CTLA4 antibody or antigen-binding fragments thereof inhibiting the binding of CTLA4 to human B7, the anti-CTLA4 antibody or antigen-binding fragments thereof binds the same epitopes as the anti-CTLA4 antibody or antigen-binding fragments thereof of any one of claims 1-13, or competes for binding human B7 with the anti-CTLA4 antibody or antigen-binding fragments thereof of any one of claims 1-13.

15. An isolated nucleic acid encoding the anti-CTLA4 antibody or antigen-binding fragments thereof of any one of claims 1-14.

16. A vector comprising the nucleic acid of claim 15.

17. A host cell comprising the nucleic acid of claim 15 or the vector of claim 16.

18. An immunoconjugate, comprising an antibody portion and a conjugation portion, wherein the antibody portion is the anti-CTLA4 antibody or antigen-binding fragments thereof of any one of claims 1-14, and the conjugation portion is a therapeutic reagent or a diagnostic reagent.

19. The immunoconjugate of claim 18, wherein the conjugation portion is selected from one or more of a radionuclide, a drug, a toxin, a cytokine, an enzyme, a fluorescein, and a biotin.

20. A pharmaceutical composition, comprising the anti-CTLA4 antibody or antigen-binding fragments thereof of any one of claims 1-14, the nucleic acid of claim 15, or the immunoconjugate of claim 18 or 19, and pharmaceutically acceptable carriers.

21. Use of the anti-CTLA4 antibody or antigen-binding fragments thereof of any one of claims 1-14, the nucleic acid of claim 15 or the immunoconjugate of claim 18 or 19 in the manufacture of medicaments for specifically binding CTLA4.

22. The use of claim 21, wherein the medicaments specifically binding CTLA are used for blocking the binding of CTLA4 to B7, down-regulating the activity or level of CTLA4, relieving the immunosuppression of CTLA4 to an organism, activating T lymphocytes, or improving the expression of IL-2 in T lymphocytes.

23. The use of claim 21, wherein the medicaments specifically binding CTLA are used for prevention and/or treatment and/or adjuvant treatment of cancers and immune system diseases.

24. The use of claim 23, wherein the cancers are selected from breast cancer, lung cancer, colorectal cancer, stomach cancer, colon cancer, esophageal cancer, ovarian cancer, cervical cancer, kidney cancer, bladder cancer, prostate cancer, pancreatic cancer, liver cancer, glioma or melanoma.

25. A method of prevention and/or treatment and/or adjuvant treatment of cancers and immune system diseases, comprising administering an effective amount of pharmaceutical compositions of claim 20 to subjects in need thereof.

26. The use of claim 25, wherein the cancers are selected from breast cancer, lung cancer, colorectal cancer, stomach cancer, colon cancer, esophageal cancer, ovarian cancer, cervical cancer, kidney cancer, bladder cancer, prostate cancer, pancreatic cancer, liver cancer, glioma or melanoma.


SEQUENCE LISTING
<110>SUZHOU GALAXY BIOPHARMA CO., LTD.
<120>CTLA4 ANTIBODY, PHARMACEUTICAL COMPOSITION AND USE THEREOF
<160> 45

<210> 1
<211>366
<212> DNA
<213>Artificial Sequence
<220>
<221> V_region
<222> (1)...(366)
<223> DNA sequence of the heavy chain variable region
<400> 1
caggtccaac tgcagcaacc tgggtctgaa ctggtgaggc ctggagcttc
agtgaagctg 60
tcctgcaagg cttctggcta cacattcacc aactactggg
tccattgggt gaaacagagg 120
cctggacaag gccttgagtg gattggaaat atttttcctg
gtagtgatac ttctaactac 180
gatgagaagt tcatcagcaa ggccacactg actgtagaca catcctccag
tgcagcctac 240
atgcagctca acagcctgac atctgaggac tctgcggtct
attactgtgc aagactcgcc 300
tactttacta actacggggg tgctatggac tactggggtc
gaggaacctc agtcaccgtc 360
tcctca 366

<210> 2
<211> 333
<212> DNA
<213>Artificial Sequence
<220>
<221> V_region
<222> (1)...(333)
<223>DNA sequence of the light chain variable region
<400> 2
gacattgtgc tgacacagtc tcctgcttcc t tagttgtatc
tctggggca g agggccacc 60
atctcatgca gggccagcaa aagtgtcagt acatctgcgt ataattatat
gcactggtac 120
caacagaaac caggacagcc acccaaactc ctcatctatc
ttgcatccaa cctagaatct 180
ggggtccctg ccaggttcag tggcagtggg tctgggacag acttcaccct
caacgtccat 240
cctgtggagg aggaggatgc tgcaacctat tactgtcagc tcagtaggga
gcttccgtac 300
acgttcggag gggggaccaa gctggaaata aaa 333

<210> 3
<211>345
<212> DNA
<213>Artificial Sequence
<220>
<221> V_region
<222> (1)...(345)
<223> DNA sequence of the heavy chain variable region
<400> 3
gaggtgaagc tggatgagac tggaggaggc ttggtgcaac ctgggaggtc
catgaaactc 60
tcctgtgttg tctctggatt cacttttagt
gacaactgga tgaactgggt ccgccagtct 120
ccagagaaag gactggagtg ggtagcacaa attagaaaca aaccttataa

ttttgaaaca 180
tattattcag attctgtgaa aggcagattc accatctcaa
gagatgattc caaaagtact 240
gtctacctgc aaatgaacaa cttaagagct gaagacatgg gtgtctatta
ctgtacgggg 300
gggtttactt actggggcca agggactctg gtcactgtct
ctgca 345

<210> 4
<211> 321
<212> DNA
<213>Artificial Sequence
<220>
<221> V_region
<222> (1)...(321)
<223> DNA sequence of the light chain variable region
<400> 4
gacatccaga tgactcagtc tccagcttca ctgtctgcat
ctgtgggaga aactgtcacc 60
atcacatgtg gagcaagtga gaatatttac ggtggtttaa attggtatca
gcggaaacag 120
ggaaaatctc ctcagctcct gatctatggt gcaaccaact tggcagaagg
catgtcatcg 180
aggttcagtg gcagtggatc tggtagacag tattctctca
agatcagtag cctgcatcct 240
gacgatgttg caacgtatta ctgtcaaaat gtgttaagta gtccgtggac
gttcggtgga 300
ggcaccaagc tggaaatcaa a 321

<210> 5
<211> 357
<212> DNA
<213>Artificial Sequence
<220>
<221> V_region
<222> (1)...(357)
<223> DNA sequence of the heavy chain variable region
<400> 5
gaggttcagct gcagcagtct gggactgtgc tggcaaggcc tggggcttcc
gtgaagatgt 60
cctgcaaggc ttctggttac agctttacca gctactggat
acactgggta aaacagaggc 120
ctggacaggg tctagaatgg attggtgctt tttatcctgg
aaatagtgat actaactaca 180
accagaactt caagggcaag gccaaattga ctgcagtcac atccgccagc
actgcctaca 240
tggagctcag cagcctgaca aatgaggact ctgcggtcta ttactgtaca
tattactacc 300
gtagtagcta tgctatggac aactggggtc aaggaacctc agtcaccgtc
tcctca 357

<210> 6
<211> 321
<212> DNA
<213>Artificial Sequence
<220>
<221> V_region
<222> (1)...(321)
<223> DNA sequence of the light chain variable region
<400>6
gacatccaga tgaaccagtc tccatccagt ctgtctgcat
cccttggaga cacaattacc 60
atcacttgcc atgccagtca gaacattaat gactggttaa gctggtacca

gcagaaacca        120
ggaaatattc        ctaaactatt        gatctataag        gcttccaact        tgcacacagg
cgtcccatca        180
aggtttagtg        gcagtggatc        tggaacaggt        ttcacattaa        ccatcagcag
cctgcagcct        240
gaagacattg        ccacttacta        ctgtcaacag        ggtcaaagtt        atcctctcac
gttcggctcg        300
gggacaaagt        tggaaataaa        a        321

<210> 7
<211>122
<212> PRT
<213>Artificial Sequence
<220>
<221> V_region
<222> (1)...(122)
<223> Amino acid sequence of the heavy chain variable region
<400> 7
Gln        Val        Gln        Leu        Gln        Gln        Pro        Gly        Ser        Glu
Leu        Val        Arg        Pro        Gly        Ala
1                                            5                                                                10
15
Ser        Val        Lys        Leu        Ser        Cys        Lys        Ala        Ser        Gly
Tyr        Thr        Phe        Thr        Asn        Tyr
                                 20                                                    25
30
Trp        Val        His        Trp        Val        Lys        Gln        Arg        Pro        Gly
Gln        Gly        Leu        Glu        Trp        Ile
                      35                                                    40
45
Gly        Asn        Ile        Phe        Pro        Gly        Ser        Asp        Thr        Ser
Asn        Tyr        Asp        Glu        Lys        Phe
           50                                                    55
60
Ile        Ser        Lys        Ala        Thr        Leu        Thr        Val        Asp        Thr
Ser        Ser        Ser        Ala        Ala        Tyr
65                                                    70
75                                                    80
Met        Gln        Leu        Asn        Ser        Leu        Thr        Ser        Glu        Asp
Ser        Ala        Val        Tyr        Tyr        Cys
                                            85                                                    90
95
Ala        Arg        Leu        Ala        Tyr        Phe        Thr        Asn        Tyr        Gly
Gly        Ala        Met        Asp        Tyr        Trp
                                 100                                                   105
110
Gly        Arg        Gly        Thr        Ser        Val        Thr        Val        Ser        Ser
     115                                                   120

<210> 8
<211> 111
<212> PRT
<213>Artificial Sequence
<220>
<221> V_region
<222> (1)...(111)
<223> Amino acid sequence of the light chain variable region
<400> 8
Asp        Ile        Val        Leu        Thr        Gln        Ser        Pro        Ala        Ser
Leu        Val        Val        Ser        Leu        Gly
1                                            5                                                                10
15
Gln        Arg        Ala        Thr        Ile        Ser        Cys        Arg        Ala        Ser

Lys Ser Val Ser Thr Ser
20 25
30
Ala Tyr Asn Tyr Met His Trp Tyr Gln Gln
Lys Pro Gly Gln Pro Pro
35 40
45
Lys Leu Leu Ile Tyr Leu Ala Ser Asn Leu
Glu Ser Gly Val Pro Ala
50 55
60
Arg Phe Ser Gly Ser Gly Ser Gly Thr Asp
Phe Thr Leu Asn Val His
65 70
75 80
Pro Val Glu Glu Glu Asp Ala Ala Thr Tyr
Tyr Cys Gln Leu Ser Arg
85 90
95
Glu Leu Pro Tyr Thr Phe Gly Gly Gly Thr
Lys Leu Glu Ile Lys
100 105
110

<210> 9
<211> 115
<212> PRT
<213>Artificial Sequence
<220>
<221> V_region
<222> (1)...(115)
<223> Amino acid sequence of the heavy chain variable region
<400> 9
Glu Val Lys Leu Asp Glu Thr Gly Gly Gly
Leu Val Gln Pro Gly Arg
1 5 10
15
Ser Met Lys Leu Ser Cys Val Val Ser Gly
Phe Thr Phe Ser Asp Asn
20 25
30
Trp Met Asn Trp Val Arg Gln Ser Pro Glu
Lys Gly Leu Glu Trp Val
35 40
45
Ala Gln Ile Arg Asn Lys Pro Tyr Asn Phe
Glu Thr Tyr Tyr Ser Asp
50 55
60
Ser Val Lys Gly Arg Phe Thr Ile Ser Arg
Asp Asp Ser Lys Ser Thr
65 70
75 80
Val Tyr Leu Gln Met Asn Asn Leu Arg Ala
Glu Asp Met Gly Val Tyr
85 90
95
Tyr Cys Thr Gly Gly Phe Thr Tyr Trp Gly
Gln Gly Thr Leu Val Thr
100 105
110
Val Ser Ala
        115

<210> 10
<211> 107
<212> PRT
<213>Artificial Sequence
<220>
<221> V_region
<222> (1)...(107)
<223>Amino acid sequence of the light chain variable region
<400> 10
Asp Ile Gln Met Thr Gln Ser Pro Ala Ser
Leu Ser Ala Ser Val Gly
1 5 10
15
Glu Thr Val Thr Ile Thr Cys Gly Ala Ser
Glu Asn Ile Tyr Gly Gly
20 25
30
Leu Asn Trp Tyr Gln Arg Lys Gln Gly Lys
Ser Pro Gln Leu Leu Ile
35 40
45
Tyr Gly Ala Thr Asn Leu Ala Glu Gly Met
Ser Ser Arg Phe Ser Gly
50 55
60
Ser Gly Ser Gly Arg Gln Tyr Ser Leu Lys
Ile Ser Ser Leu His Pro
65 70
75 80
Asp Asp Val Ala Thr Tyr Tyr Cys Gln Asn
Val Leu Ser Ser Pro Trp
85 90
95
Thr Phe Gly Gly Gly Thr Lys Leu Glu Ile
Lys
100 105

<210> 11
<211> 119
<212> PRT
<213>Artificial Sequence
<220>
<221> V_region
<222> (1)...(119)
<223> Amino acid sequence of the heavy chain variable region
<400> 11
Glu Val Gln Leu Gln Gln Ser Gly Thr Val
Leu Ala Arg Pro Gly Ala
1 5 10
15
Ser Val Lys Met Ser Cys Lys Ala Ser Gly
Tyr Ser Phe Thr Ser Tyr
20 25
30
Trp Ile His Trp Val Lys Gln Arg Pro Gly
Gln Gly Leu Glu Trp Ile
35 40
45
Gly Ala Phe Tyr Pro Gly Asn Ser Asp Thr
Asn Tyr Asn Gln Asn Phe
50 55

60
Lys Gly Lys Ala Lys Leu Thr Ala Val Thr
Ser Ala Ser Thr Ala Tyr
65 70
75 80
Met Glu Leu Ser Ser Leu Thr Asn Glu Asp
Ser Ala Val Tyr Tyr Cys
85 90
95
Thr Tyr Tyr Tyr Arg Ser Ser Tyr Ala Met
Asp Asn Trp Gly Gln Gly
100 105
110
Thr Ser Val Thr Val Ser Ser
        115

<210> 12
<211> 107
<212> PRT
<213>Artificial Sequence
<220>
<221> V_region
<222> (1)...(107)
<223> Amino acid sequence of the light chain variable region
<400> 12
Asp Ile Gln Met Asn Gln Ser Pro Ser Ser
Leu Ser Ala Ser Leu Gly
1 5 10
15
Asp Thr Ile Thr Ile Thr Cys His Ala Ser
Gln Asn Ile Asn Asp Trp
20 25
30
Leu Ser Trp Tyr Gln Gln Lys Pro Gly Asn
Ile Pro Lys Leu Leu Ile
35 40
45
Tyr Lys Ala Ser Asn Leu His Thr Gly Val
Pro Ser Arg Phe Ser Gly
50 55
60
Ser Gly Ser Gly Thr Gly Phe Thr Leu Thr
Ile Ser Ser Leu Gln Pro
65 70
75 80
Glu Asp Ile Ala Thr Tyr Tyr Cys Gln Gln
Gly Gln Ser Tyr Pro Leu
85 90
95
Thr Phe Gly Ser Gly Thr Lys Leu Glu Ile
Lys
100 105

<210> 13
<211> 10
<212> PRT
<213>Artificial Sequence
<220>
<221> misc_feature
<222> (1)...(10)
<223> The amino acid sequence of the heavy chain CDR1
<400> 13
Gly Tyr Thr Phe Thr Asn Tyr Trp Val His

1                                   5                                       10

<210> 14
<211> 17
<212> PRT
<213>Artificial Sequence
<220>
<221> misc_feature
<222> (1)...(17)
<223> The amino acid sequence of the heavy chain CDR2
<400> 14
Asn     Ile     Phe     Pro     Gly     Ser     Asp     Thr     Ser     Asn
Tyr     Asp     Glu     Lys     Phe     Ile
1                                   5                                       10
15
Ser

<210> 15
<211> 15
<212> PRT
<213>Artificial Sequence
<220>
<221> misc_feature
<222> (1)...(15)
<223> The amino acid sequence of the heavy chain CDR3
<400> 15
Ala     Arg     Leu     Ala     Tyr     Phe     Thr     Asn     Tyr     Gly
Gly     Ala     Met     Asp     Tyr
1                                   5                                       10
15

<210> 16
<211> 15
<212> PRT
<213>Artificial Sequence
<220>
<221> misc_feature
<222> (1)...(15)
<223> The amino acid sequence of the light chain CDR1
<400> 16
Arg     Ala     Ser     Lys     Ser     Val     Ser     Thr     Ser     Ala
Tyr     Asn     Tyr     Met     His
1                                   5                                       10
15

<210> 17
<211> 7
<212> PRT
<213>Artificial Sequence
<220>
<221> misc_feature
<222> (1)...(7)
<223> The amino acid sequence of the light chain CDR2
<400> 17
Leu     Ala     Ser     Asn     Leu     Glu     Ser
1                                   5

<210> 18
<211> 9
<212> PRT
<213>Artificial Sequence

<220>
<221> misc_feature
<222> (1)...(9)
<223> The amino acid sequence of the light chain CDR3
<400> 18
Gln Leu Ser Arg Glu Leu Pro Tyr Thr
1               5

<210> 19
<211> 10
<212> PRT
<213>Artificial Sequence
<220>
<221> misc_feature
<222> (1)...(10)
<223> The amino acid sequence of the heavy chain CDR1
<400> 19
Gly Phe Thr Phe Ser Asp Asn Trp Met Asn

1               5                   10


<210> 20
<211> 19
<212> PRT
<213>Artificial Sequence
<220>
<221> misc_feature
<222> (1)...(19)
<223> The amino acid sequence of the heavy chain CDR2
<400> 20
Gln Ile Arg Asn Lys Pro Tyr Asn Phe Glu
Thr Tyr Tyr Ser Asp Ser
1 5 10
15
Val Lys Gly

<210> 21
<211> 6
<212> PRT
<213>Artificial Sequence
<220>
<221> misc_feature
<222> (1)...(6)
<223> The amino acid sequence of the heavy chain CDR3
<400> 21
Thr Gly Gly Phe Thr Tyr
1               5

<210> 22
<211> 11
<212> PRT
<213>Artificial Sequence
<220>
<221> misc_feature
<222> (1)...(11)
<223> The amino acid sequence of the light chain CDR1
<400> 22
Gly Ala Ser Glu Asn Ile Tyr Gly Gly Leu
Asn
1 5 10

<210> 23
<211>7
<212> PRT
<213>Artificial Sequence
<220>
<221> misc_feature
<222> (1)...(7)
<223> The amino acid sequence of the light chain CDR2
<400> 23
Gly     Ala     Thr     Asn     Leu     Ala     Glu
1                               5

<210> 24
<211> 9
<212> PRT
<213>Artificial Sequence
<220>
<221> misc_feature
<222> (1)...(9)
<223> The amino acid sequence of the light chain CDR3
<400> 24
Gln     Asn     Val     Leu     Ser     Ser     Pro     Trp     Thr
1                               5

<210> 25
<211> 10
<212> PRT
<213>Artificial Sequence
<220>
<221> misc_feature
<222> (1)...(10)
<223> The amino acid sequence of the heavy chain CDR1
<400> 25
Gly     Tyr     Ser     Phe     Thr     Ser     Tyr     Trp     Ile     His

1                               5                                       10


<210> 26
<211> 17
<212> PRT
<213>Artificial Sequence
<220>
<221> misc_feature
<222> (1)...(17)
<223> The amino acid sequence of the heavy chain CDR2
<400> 26
Ala     Phe     Tyr     Pro     Gly     Asn     Ser     Asp     Thr     Asn
Tyr     Asn     Gln     Asn     Phe     Lys
1                               5                                       10
15
Gly

<210> 27
<211> 12
<212> PRT
<213>Artificial Sequence
<220>
<221> misc_feature
<222> (1)...(12)
<223> The amino acid sequence of the heavy chain CDR3
<400> 27
Thr     Tyr     Tyr     Tyr     Arg     Ser     Ser     Tyr     Ala     Met

Asp Asn
1 5 10

<210> 28
<211> 11
<212> PRT
<213>Artificial Sequence
<220>
<221> misc_feature
<222> (1)...(11)
<223> The amino acid sequence of the light chain CDR1
<400> 28
His Ala Ser Gln Asn Ile Asn Asp Trp Leu
Ser
1 5 10

<210> 29
<211>7
<212> PRT
<213>Artificial Sequence
<220>
<221> misc_feature
<222> (1)...(7)
<223> The amino acid sequence of the light chain CDR2
<400> 29
Lys Ala Ser Asn Leu His Thr
1               5

<210> 30
<211> 9
<212> PRT
<213>Artificial Sequence
<220>
<221> misc_feature
<222> (1)...(9)
<223> The amino acid sequence of the light chain CDR3
<400> 30
Gln Gln Gly Gln Ser Tyr Pro Leu Thr
1               5

<210> 31
<211> 366
<212> DNA
<213>Artificial Sequence
<220>
<221> V_region
<222> (1)...(366)
<223> DNA sequence of the heavy chain variable region
<400> 31
gaggttcaac ttgttgaatc tgggggcgga ctggtccagc
caggaggaag cctcaggctc 60
tcatgcgccg ccagtggtta cacctttacc aactactggg tacactgggt
gcgccaggcc 120
cctggaaaag gattggaatg gattggcaac atatttcctg
ggtcagacac aagcaactat 180
gatgaaaaat ttatcagtaa agctacactt tccgtggaca ccagtaaaaa
tactgcttat 240
cttcagatga actccttgcg tgcagaggac accgctgtct attattgtgc
tcgcctggca 300
tactttacaa attacggcgg agccatggac tactggggtc aaggtacact
ggtcactgtc 360

tcgagc 366

<210> 32
<211> 366
<212> DNA
<213>Artificial Sequence
<220>
<221> V_region
<222> (1)...(366)
<223> DNA sequence of the heavy chain variable region
<400> 32
gaggttcaac ttgttgaatc tgggggcgga ctggtccagc
caggaggaag cctcaggctc 60
Tcatgcgccg ccagtggtta cacctttacc aactactggg tacactgggt
gcgccaggcc 120
cctggaaaag gattggaatg ggtgggcaac atatttcctg
ggtcagacac aagcaactat 180
gatgaaaaat ttatcagtcg atttacaata tccgtggaca ccagtaaaaa
tactttgtat 240
cttcagatga actccttgcg tgcagaggac accgctgtct attattgtgc
tcgcctggca 300
tactttacaa attacggcgg agccatggac tactggggtc aaggtacact
ggtcactgtc 360
tcgagc 366

<210> 33
<211> 366
<212> DNA
<213>Artificial Sequence
<220>
<221> V_region
<222> (1)...(366)
<223> DNA sequence of the heavy chain variable region
<400> 33
gaggttcaac ttgttgaatc tgggggcgga ctggtccagc
caggaggaag cctcaggctc 60
tcatgcgccg ccagtggtta cacctttacc aactactggg tacactgggt
gcgccaggcc 120
cctggaaaag gattggaatg ggtgggcaac atatttcctg
ggtcagacac aagcaactat 180
gatgaaaaat ttatcagtcg gttcacactt tccgtggaca
ccagtaaaaa tactgcttat 240
cttcagatga actccttgcg tgcagaggac accgctgtct attattgtgc
tcgcctggca 300
tactttacaa attacggcgg agccatggac tactggggtc aaggtacact
ggtcactgtc 360
tcgagc 366

<210> 34
<211> 333
<212> DNA
<213>Artificial Sequence
<220>
<221> V_region
<222> (1)...(333)
<223> DNA sequence of the light chain variable region
<400> 34
Gacattgttc tcacacagag cccttctagc cttgtagcct
ccgtagggga caggtaaca 60
ataagttgtc gagctagtaa gtcagtttca acctctgcct
acaattacat gcactggtat 120
cagcaaaaac ccggaaaggc tcctaagctc ttgatatatc
tggcctcaaa tctggagtca 180

ggagttccat ccagatttag cggcagcggg tctggaaccg acttcaccct
cacaatctcc 240
ccttgcaac ccgaggactt cgccacctat tactgccaac
tctcccgaga attgccatat 300
acattcggtc aaggaaccaa agttgaaata aag 333

<210> 35
<211> 333
<212> DNA
<213>Artificial Sequence
<220>
<221> V_region
<222> (1)...(333)
<223> DNA sequence of the light chain variable region
<400> 35
gacattcaac tgacacagag cccttctagc ctttcagcct
ccgtagggga cagggtaaca 60
ataacatgtc gagctagtaa gtcagtttca acctctgcct
acaattacat gcactggtat 120
cagcaaaaac ccggaaaggc tcctaagctc ttgatatatc
tggcctcaaa tctggagtca 180
ggagttccat ccagatttag cggcagcggg tctggaaccg acttcaccct
cacaatctcc 240
tccttgcaac ccgaggactt cgccacctat tactgccaac tctcccgaga
attgccatat 300
acattcggtc aaggaaccaa agttgaaata aag 333

<210> 36
<211> 122
<212>DNA
<213>Artificial Sequence
<220>
<221> V_region
<222> (1)...(122)
<223> Amino acid sequence of the heavy chain variable region
<400> 36
Glu Val Gln Leu Val Glu Ser Gly Gly Gly
Leu Val Gln Pro Gly Gly
1 5 10
15
Ser Leu Arg Leu Ser Cys Ala Ala Ser Gly
Tyr Thr Phe Thr Asn Tyr
20 25
30
Trp Val His Trp Val Arg Gln Ala Pro Gly
Lys Gly Leu Glu Trp Ile
35 40
45
Gly Asn Ile Phe Pro Gly Ser Asp Thr Ser
Asn Tyr Asp Glu Lys Phe
50 55
60
Ile Ser Lys Ala Thr Leu Ser Val Asp Thr
Ser Lys Asn Thr Ala Tyr
65 70
75 80
Leu Gln Met Asn Ser Leu Arg Ala Glu Asp
Thr Ala Val Tyr Tyr Cys
85 90
95
Ala Arg Leu Ala Tyr Phe Thr Asn Tyr Gly
Gly Ala Met Asp Tyr Trp
100 105

110
Gly Gln Gly Thr Leu Val Thr Val Ser Ser
        115                 120

<210> 37
<211> 122
<212> DNA
<213>Artificial Sequence
<220>
<221> V_region
<222> (1)...(122)
<223> Amino acid sequence of the heavy chain variable region
<400> 37
Glu Val Gln Leu Val Glu Ser Gly Gly Gly
Leu Val Gln Pro Gly Gly
1 5 10
15
Ser Leu Arg Leu Ser Cys Ala Ala Ser Gly
Tyr Thr Phe Thr Asn Tyr
20 25
30
Trp Val His Trp Val Arg Gln Ala Pro Gly
Lys Gly Leu Glu Trp Val
35 40
45
Gly Asn Ile Phe Pro Gly Ser Asp Thr Ser
Asn Tyr Asp Glu Lys Phe
50 55
60
Ile Ser Arg Phe Thr Ile Ser Val Asp Thr
Ser Lys Asn Thr Leu Tyr
65 70
75 80
Leu Gln Met Asn Ser Leu Arg Ala Glu Asp
Thr Ala Val Tyr Tyr Cys
85 90
95
Ala Arg Leu Ala Tyr Phe Thr Asn Tyr Gly
Gly Ala Met Asp Tyr Trp
100 105
110
Gly Gln Gly Thr Leu Val Thr Val Ser Ser
        115                 120

<210> 38
<211> 122
<212> DNA
<213>Artificial Sequence
<220>
<221> V_region
<222> (1)...(122)
<223> Amino acid sequence of the heavy chain variable region
<400> 38
Glu Val Gln Leu Val Glu Ser Gly Gly Gly
Leu Val Gln Pro Gly Gly
1 5 10
15
Ser Leu Arg Leu Ser Cys Ala Ala Ser Gly
Tyr Thr Phe Thr Asn Tyr
20 25
30

Trp Val His Trp Val Arg Gln Ala Pro Gly
Lys Gly Leu Glu Trp Val
35 40
45
Gly Asn Ile Phe Pro Gly Ser Asp Thr Ser
Asn Tyr Asp Glu Lys Phe
50 55
60
Ile Ser Arg Phe Thr Leu Ser Val Asp Thr
Ser Lys Asn Thr Ala Tyr
65 70
75 80
Leu Gln Met Asn Ser Leu Arg Ala Glu Asp
Thr Ala Val Tyr Tyr Cys
85 90
95
Ala Arg Leu Ala Tyr Phe Thr Asn Tyr Gly
Gly Ala Met Asp Tyr Trp
100 105
110
Gly Gln Gly Thr Leu Val Thr Val Ser Ser

        115                 120

<210> 39
<211> 111
<212> DNA
<213>Artificial Sequence
<220>
<221> V_region
<222> (1)...(111)
<223> Amino acid sequence of the light chain variable region
<400> 39
Asp Ile Val Leu Thr Gln Ser Pro Ser Ser
Leu Val Ala Ser Val Gly
1 5 10
15
Asp Arg Val Thr Ile Ser Cys Arg Ala Ser
Lys Ser Val Ser Thr Ser
20 25
30
Ala Tyr Asn Tyr Met His Trp Tyr Gln Gln
Lys Pro Gly Lys Ala Pro
35 40
45
Lys Leu Leu Ile Tyr Leu Ala Ser Asn Leu
Glu Ser Gly Val Pro Ser
50 55
60
Arg Phe Ser Gly Ser Gly Ser Gly Thr Asp
Phe Thr Leu Thr Ile Ser
65 70
75 80
Ser Leu Gln Pro Glu Asp Phe Ala Thr Tyr
Tyr Cys Gln Leu Ser Arg
85 90
95
Glu Leu Pro Tyr Thr Phe Gly Gln Gly Thr
Lys Val Glu Ile Lys
100 105
110

<210> 40

<211> 111
<212> DNA
<213>Artificial Sequence
<220>
<221> V_region
<222> (1)...(111)
<223> Amino acid sequence of the light chain variable region
<400> 40
Asp Ile Gln Leu Thr Gln Ser Pro Ser Ser
Leu Ser Ala Ser Val Gly
1 5 10
15
Asp Arg Val Thr Ile Thr Cys Arg Ala Ser
Lys Ser Val Ser Thr Ser
20 25
30
Ala Tyr Asn Tyr Met His Trp Tyr Gln Gln
Lys Pro Gly Lys Ala Pro
35 40
45
Lys Leu Leu Ile Tyr Leu Ala Ser Asn Leu
Glu Ser Gly Val Pro Ser
50 55
60
Arg Phe Ser Gly Ser Gly Ser Gly Thr Asp
Phe Thr Leu Thr Ile Ser
65 70
75 80
Ser Leu Gln Pro Glu Asp Phe Ala Thr Tyr
Tyr Cys Gln Leu Ser Arg
85 90
95
Glu Leu Pro Tyr Thr Phe Gly Gln Gly Thr
Lys Val Glu Ile Lys
100 105
110

<210> 41
<211> 452
<212> DNA
<213>Artificial Sequence
<220>
<221> misc_feature
<222> (1)...(452)
<223>Amino acid sequence of full-length heavy chain
<400> 41
Glu Val Gln Leu Val Glu Ser Gly Gly Gly
Leu Val Gln Pro Gly Gly
1 5 10
15
Ser Leu Arg Leu Ser Cys Ala Ala Ser Gly
Tyr Thr Phe Thr Asn Tyr
20 25
30
Trp Val His Trp Val Arg Gln Ala Pro Gly
Lys Gly Leu Glu Trp Ile
35 40
45
Gly Asn Ile Phe Pro Gly Ser Asp Thr Ser
Asn Tyr Asp Glu Lys Phe
50 55
60
Ile Ser Lys Ala Thr Leu Ser Val Asp Thr

Ser Lys Asn Thr Ala Tyr
65 70
75 80
Leu Gln Met Asn Ser Leu Arg Ala Glu Asp
Thr Ala Val Tyr Tyr Cys
85 90
95
Ala Arg Leu Ala Tyr Phe Thr Asn Tyr Gly
Gly Ala Met Asp Tyr Trp
100 105
110
Gly Gln Gly Thr Leu Val Thr Val Ser Ser
Ala Ser Thr Lys Gly Pro
115 120
125
Ser Val Phe Pro Leu Ala Pro Ser Ser Lys
Ser Thr Ser Gly Gly Thr
130 135
140
Ala Ala Leu Gly Cys Leu Val Lys Asp Tyr
Phe Pro Glu Pro Val Thr
145 150
155 160
Val Ser Trp Asn Ser Gly Ala Leu Thr Ser
Gly Val His Thr Phe Pro
165 170
175
Ala Val Leu Gln Ser Ser Gly Leu Tyr Ser
Leu Ser Ser Val Val Thr
180 185
190
Val Pro Ser Ser Ser Leu Gly Thr Gln Thr
Tyr Ile Cys Asn Val Asn
195 200
205
His Lys Pro Ser Asn Thr Lys Val Asp Lys
Arg Val Glu Pro Lys Ser
210 215
220
Cys Asp Lys Thr His Thr Cys Pro Pro Cys
Pro Ala Pro Glu Leu Leu
225 230
235 240
Gly Gly Pro Ser Val Phe Leu Phe Pro Pro
Lys Pro Lys Asp Thr Leu
245 250
255
Met Ile Ser Arg Thr Pro Glu Val Thr Cys
Val Val Val Asp Val Ser
260 265
270
His Glu Asp Pro Glu Val Lys Phe Asn Trp
Tyr Val Asp Gly Val Glu
275 280
285
Val His Asn Ala Lys Thr Lys Pro Arg Glu
Glu Gln Tyr Asn Ser Thr
290 295
300
Tyr Arg Val Val Ser Val Leu Thr Val Leu
His Gln Asp Trp Leu Asn
305 310
315 320

Gly Lys Glu Tyr Lys Cys Lys Val Ser Asn
Lys Ala Leu Pro Ala Pro
325 330
335
Ile Glu Lys Thr Ile Ser Lys Ala Lys Gly
Gln Pro Arg Glu Pro Gln
340 345
350
Val Tyr Thr Leu Pro Pro Ser Arg Asp Glu
Leu Thr Lys Asn Gln Val
355 360
365
Ser Leu Thr Cys Leu Val Lys Gly Phe Tyr
Pro Ser Asp Ile Ala Val
370 375
380
Glu Trp Glu Ser Asn Gly Gln Pro Glu Asn
Asn Tyr Lys Thr Thr Pro
385 390
395 400
Pro Val Leu Asp Ser Asp Gly Ser Phe Phe
Leu Tyr Ser Lys Leu Thr
405 410
415
Val Asp Lys Ser Arg Trp Gln Gln Gly Asn
Val Phe Ser Cys Ser Val
420 425
430
Met His Glu Ala Leu His Asn His Tyr Thr
Gln Lys Ser Leu Ser Leu
435 440
445
Ser Pro Gly Lys
    450

<210> 42
<211> 452
<212> DNA
<213>Artificial Sequence
<220>
<221> misc_feature
<222> (1)...(452)
<223>Amino acid sequence of full-length heavy chain
<400> 42
Glu Val Gln Leu Val Glu Ser Gly Gly Gly
Leu Val Gln Pro Gly Gly
1 5 10
15
Ser Leu Arg Leu Ser Cys Ala Ala Ser Gly
Tyr Thr Phe Thr Asn Tyr
20 25
30
Trp Val His Trp Val Arg Gln Ala Pro Gly
Lys Gly Leu Glu Trp Val
35 40
45
Gly Asn Ile Phe Pro Gly Ser Asp Thr Ser
Asn Tyr Asp Glu Lys Phe
50 55
60
Ile Ser Arg Phe Thr Ile Ser Val Asp Thr
Ser Lys Asn Thr Leu Tyr
65 70

75                                      80
Leu     Gln     Met     Asn     Ser     Leu     Arg     Ala     Glu     Asp
Thr     Ala     Val     Tyr     Tyr     Cys
                                85                                      90
95
Ala     Arg     Leu     Ala     Tyr     Phe     Thr     Asn     Tyr     Gly
Gly     Ala     Met     Asp     Tyr     Trp
                        100                                     105
110
Gly     Gln     Gly     Thr     Leu     Val     Thr     Val     Ser     Ser
Ala     Ser     Thr     Lys     Gly     Pro
                115                                     120
125
Ser     Val     Phe     Pro     Leu     Ala     Pro     Ser     Ser     Lys
Ser     Thr     Ser     Gly     Gly     Thr
        130                                     135
140
Ala     Ala     Leu     Gly     Cys     Leu     Val     Lys     Asp     Tyr
Phe     Pro     Glu     Pro     Val     Thr
145                                     150
155                                     160
Val     Ser     Trp     Asn     Ser     Gly     Ala     Leu     Thr     Ser
Gly     Val     His     Thr     Phe     Pro
                                165                                     170
175
Ala     Val     Leu     Gln     Ser     Ser     Gly     Leu     Tyr     Ser
Leu     Ser     Ser     Val     Val     Thr
                        180                                     185
190
Val     Pro     Ser     Ser     Ser     Leu     Gly     Thr     Gln     Thr
Tyr     Ile     Cys     Asn     Val     Asn
                195                                     200
205
His     Lys     Pro     Ser     Asn     Thr     Lys     Val     Asp     Lys
Arg     Val     Glu     Pro     Lys     Ser
        210                                     215
220
Cys     Asp     Lys     Thr     His     Thr     Cys     Pro     Pro     Cys
Pro     Ala     Pro     Glu     Leu     Leu
225                                     230
235                                     240
Gly     Gly     Pro     Ser     Val     Phe     Leu     Phe     Pro     Pro
Lys     Pro     Lys     Asp     Thr     Leu
                                245                                     250
255
Met     Ile     Ser     Arg     Thr     Pro     Glu     Val     Thr     Cys
Val     Val     Val     Asp     Val     Ser
                        260                                     265
270
His     Glu     Asp     Pro     Glu     Val     Lys     Phe     Asn     Trp
Tyr     Val     Asp     Gly     Val     Glu
                275                                     280
285
Val     His     Asn     Ala     Lys     Thr     Lys     Pro     Arg     Glu
Glu     Gln     Tyr     Asn     Ser     Thr
        290                                     295
300
Tyr     Arg     Val     Val     Ser     Val     Leu     Thr     Val     Leu
His     Gln     Asp     Trp     Leu     Asn
305                                     310
315                                     320
Gly     Lys     Glu     Tyr     Lys     Cys     Lys     Val     Ser     Asn
Lys     Ala     Leu     Pro     Ala     Pro

325 330
335
Ile Glu Lys Thr Ile Ser Lys Ala Lys Gly
Gln Pro Arg Glu Pro Gln
340 345
350
Val Tyr Thr Leu Pro Pro Ser Arg Asp Glu
Leu Thr Lys Asn Gln Val
355 360
365
Ser Leu Thr Cys Leu Val Lys Gly Phe Tyr
Pro Ser Asp Ile Ala Val
370 375
380
Glu Trp Glu Ser Asn Gly Gln Pro Glu Asn
Asn Tyr Lys Thr Thr Pro
385 390
395 400
Pro Val Leu Asp Ser Asp Gly Ser Phe Phe
Leu Tyr Ser Lys Leu Thr
405 410
415
Val Asp Lys Ser Arg Trp Gln Gln Gly Asn
Val Phe Ser Cys Ser Val
420 425
430
Met His Glu Ala Leu His Asn His Tyr Thr
Gln Lys Ser Leu Ser Leu
435 440
445
Ser Pro Gly Lys
    450

<210> 43
<211> 452
<212> DNA
<213>Artificial Sequence
<220>
<221> misc_feature
<222> (1)...(452)
<223>Amino acid sequence of full-length heavy chain
<400> 43
Glu Val Gln Leu Val Glu Ser Gly Gly Gly
Leu Val Gln Pro Gly Gly
1 5 10
15
Ser Leu Arg Leu Ser Cys Ala Ala Ser Gly
Tyr Thr Phe Thr Asn Tyr
20 25
30
Trp Val His Trp Val Arg Gln Ala Pro Gly
Lys Gly Leu Glu Trp Val
35 40
45
Gly Asn Ile Phe Pro Gly Ser Asp Thr Ser
Asn Tyr Asp Glu Lys Phe
50 55
60
Ile Ser Arg Phe Thr Leu Ser Val Asp Thr
Ser Lys Asn Thr Ala Tyr
65 70
75 80
Leu Gln Met Asn Ser Leu Arg Ala Glu Asp

Thr Ala Val Tyr Tyr Cys
85 90
95
Ala Arg Leu Ala Tyr Phe Thr Asn Tyr Gly
Gly Ala Met Asp Tyr Trp
100 105
110
Gly Gln Gly Thr Leu Val Thr Val Ser Ser
Ala Ser Thr Lys Gly Pro
115 120
125
Ser Val Phe Pro Leu Ala Pro Ser Ser Lys
Ser Thr Ser Gly Gly Thr
130 135
140
Ala Ala Leu Gly Cys Leu Val Lys Asp Tyr
Phe Pro Glu Pro Val Thr
145 150
155 160
Val Ser Trp Asn Ser Gly Ala Leu Thr Ser
Gly Val His Thr Phe Pro
165 170
175
Ala Val Leu Gln Ser Ser Gly Leu Tyr Ser
Leu Ser Ser Val Val Thr
180 185
190
Val Pro Ser Ser Ser Leu Gly Thr Gln Thr
Tyr Ile Cys Asn Val Asn
195 200
205
His Lys Pro Ser Asn Thr Lys Val Asp Lys Arg
Val Glu Pro Lys Ser
210 215
220
Cys Asp Lys Thr His Thr Cys Pro Pro Cys Pro
Ala Pro Glu Leu Leu
225 230
235 240
Gly Gly Pro Ser Val Phe Leu Phe Pro Pro
Lys Pro Lys Asp Thr Leu
245 250
255
Met Ile Ser Arg Thr Pro Glu Val Thr Cys
Val Val Val Asp Val Ser
260 265
270
His Glu Asp Pro Glu Val Lys Phe Asn Trp
Tyr Val Asp Gly Val Glu
275 280
285
Val His Asn Ala Lys Thr Lys Pro Arg Glu
Glu Gln Tyr Asn Ser Thr
290 295
300
Tyr Arg Val Val Ser Val Leu Thr Val Leu
His Gln Asp Trp Leu Asn
305 310
315 320
Gly Lys Glu Tyr Lys Cys Lys Val Ser Asn
Lys Ala Leu Pro Ala Pro
325 330
335

Ile Glu Lys Thr Ile Ser Lys Ala Lys Gly
Gln Pro Arg Glu Pro Gln
340 345
350
L Val Tyr Thr Leu Pro Pro Ser Arg Asp Glu
Leu Thr ys Asn Gln Val
355 360
365
Ser Leu Thr Cys Leu Val Lys Gly Phe Tyr
Pro Ser Asp Ile Ala Val
370 375
380
Glu Trp Glu Ser Asn Gly Gln Pro Glu Asn
Asn Tyr Lys Thr Thr Pro
385 390
395 400
Pro Val Leu Asp Ser Asp Gly Ser Phe Phe
Leu Tyr Ser Lys Leu Thr
405 410
415
Val Asp Lys Ser Arg Trp Gln Gln Gly Asn
Val Phe Ser Cys Ser Val
420 425
430
Met His Glu Ala Leu His Asn His Tyr Thr
Gln Lys Ser Leu Ser Leu
435 440
445
Ser Pro Gly Lys
450

<210> 44
<211> 218
<212> DNA
<213>Artificial Sequence
<220>
<221> misc_feature
<222> (1)...(218)
<223>Amino acid sequence of full-length light chain
<400> 44
Asp Ile Val Leu Thr Gln Ser Pro Ser Ser
Leu Val Ala Ser Val Gly
1 5 10
15
Asp Arg Val Thr Ile Ser Cys Arg Ala Ser
Lys Ser Val Ser Thr Ser
20 25
30
Ala Tyr Asn Tyr Met His Trp Tyr Gln Gln
Lys Pro Gly Lys Ala Pro
35 40
45
Lys Leu Leu Ile Tyr Leu Ala Ser Asn Leu
Glu Ser Gly Val Pro Ser
50 55
60
Arg Phe Ser Gly Ser Gly Ser Gly Thr Asp
Phe Thr Leu Thr Ile Ser
65 70
75 80
Ser Leu Gln Pro Glu Asp Phe Ala Thr Tyr
Tyr Cys Gln Leu Ser Arg
85 90

95
Glu Leu Pro Tyr Thr Phe Gly Gln Gly Thr
Lys Val Glu Ile Lys Arg
100 105
110
Thr Val Ala Ala Pro Ser Val Phe Ile Phe
Pro Pro Ser Asp Glu Gln
115 120
125
Leu Lys Ser Gly Thr Ala Ser Val Val Cys
Leu Leu Asn Asn Phe Tyr
130 135
140
Pro Arg Glu Ala Lys Val Gln Trp Lys Val
Asp Asn Ala Leu Gln Ser
145 150
155 160
Gly Asn Ser Gln Glu Ser Val Thr Glu Gln
Asp Ser Lys Asp Ser Thr
165 170
175
Tyr Ser Leu Ser Ser Thr Leu Thr Leu Ser
Lys Ala Asp Tyr Glu Lys
180 185
190
His Lys Val Tyr Ala Cys Glu Val Thr His
Gln Gly Leu Ser Ser Pro
195 200
205
Val Thr Lys Ser Phe Asn Arg Gly Glu Cys

    210                 215

<210> 45
<211> 218
<212> DNA
<213>Artificial Sequence
<220>
<221> misc_feature
<222> (1)...(218)
<223>Amino acid sequence of full-length light chain
<400> 45
Asp Ile Gln Leu Thr Gln Ser Pro Ser Ser
Leu Ser Ala Ser Val Gly
1 5 10
15
Asp Arg Val Thr Ile Thr Cys Arg Ala Ser
Lys Ser Val Ser Thr Ser
20 25
30
Ala Tyr Asn Tyr Met His Trp Tyr Gln Gln
Lys Pro Gly Lys Ala Pro
35 40
45
Lys Leu Leu Ile Tyr Leu Ala Ser Asn Leu
Glu Ser Gly Val Pro Ser
50 55
60
Arg Phe Ser Gly Ser Gly Ser Gly Thr Asp
Phe Thr Leu Thr Ile Ser
65 70
75 80

Ser Leu Gln Pro Glu Asp Phe Ala Thr Tyr
Tyr Cys Gln Leu Ser Arg
85 90
95
Glu Leu Pro Tyr Thr Phe Gly Gln Gly Thr
Lys Val Glu Ile Lys Arg
100 105
110
Thr Val Ala Ala Pro Ser Val Phe Ile Phe
Pro Pro Ser Asp Glu Gln
115 120
125
Leu Lys Ser Gly Thr Ala Ser Val Val Cys
Leu Leu Asn Asn Phe Tyr
130 135
140
Pro Arg Glu Ala Lys Val Gln Trp Lys Val
Asp Asn Ala Leu Gln Ser
145 150
155 160
Gly Asn Ser Gln Glu Ser Val Thr Glu Gln
Asp Ser Lys Asp Ser Thr
165 170
175
Tyr Ser Leu Ser Ser Thr Leu Thr Leu Ser
Lys Ala Asp Tyr Glu Lys
180 185
190
His Lys Val Tyr Ala Cys Glu Val Thr His
Gln Gly Leu Ser Ser Pro
195 200
205
Val Thr Lys Ser Phe Asn Arg Gly Glu Cys

    210                 215